# FASCICULE DE BREVET EUROPEEN

(11) **EP 3 989 943 B1**
(45) Date de publication et mention de la délivrance du brevet: **20.05.2026**
(21) Numéro de dépôt: 20735139.6
(22) Date de dépôt: 24.06.2020
(51) Int. Cl.: A61K 9/16, A61K 31/19, A61K 31/34, A61K 31/45

(54) **COMPOSITION COMPRENANT AU MOINS UN TRITERPÈNE ET/OU AU MOINS UN TRITERPÉNOÏDE ET/OU AU MOINS UNE DE LEURS FORMES GLYCOSYLÉES**
ZUSAMMENSETZUNG MIT MINDESTENS EINEM TRITERPEN UND/ODER MINDESTENS EINEM TRITERPENOID UND/ODER MINDESTENS EINER DER GLYKOSYLIERTEN FORMEN DAVON
COMPOSITION COMPRISING AT LEAST ONE TRITERPENE AND/OR AT LEAST ONE TRITERPENOID AND/OR AT LEAST ONE OF THE GLYCOSYLATED FORMS THEREOF

(30) Priorité: 28.06.2019 BE 201905419; 10.07.2019 BE 201905448; 17.04.2020 BE 202005254
(43) Date de publication de la demande: 04.05.2022
(73) Titulaire: Tilman, 5377 Baillonville (BE); Eleonor, 1410 Waterloo (BE)
(72) Inventeur: DIERCKXSENS, Yvan, 3090 Overijse (BE); MEINGUET, Céline, 5590 Ciney (BE); TILMAN, Jean-Noël, 4920 Ernonheid (BE); WÉRY, Benoît, 4121 Neupré (BE); LOIRA-PASTORIZA, Cristina, 1120 Bruxelles (BE); MARTINEZ-TOVAR, Daniela, 94500 Champigny-sur-Marne (FR); PRIEM, Fabian, 1410 Waterloo (BE)
(74) Mandataire: Gevers Patents
(86) Numéro de dépôt international: PCT/EP2020/067742
(87) Numéro de publication internationale: WO 2020/260413

(56) Documents cités:
- EP-A1- 0 599 282
- EP-A1- 1 391 426
- WO-A1-2019/038430
- US-A- 5 260 074
- US-A1- 2018 085 316
- US-A1- 2018 263 270
- DATABASE MEDLINE [online] US NATIONAL LIBRARY OF MEDICINE (NLM), BETHESDA, MD, US; 2015, TACKENBERG MARKUS W ET AL: "Encapsulation of orange terpenes investigating a plasticisation extrusion process.", Database accession no. NLM26052721
- TACKENBERG MARKUS W ET AL: "Encapsulation of orange terpenes investigating a plasticisation extrusion process.", JOURNAL OF MICROENCAPSULATION 2015, vol. 32, no. 4, 2015, pages 408 - 417, ISSN: 1464-5246
- GAO NANNAN ET AL: "Application of hot melt extrusion to enhance the dissolution and oral bioavailability of oleanolic acid", ASIAN JOURNAL OF PHARMACEUTICAL SCIENCES, vol. 12, no. 1, 1 January 2017 (2017-01-01), NL, pages 66 - 72, XP093172750, ISSN: 1818-0876, DOI: 10.1016/j.ajps.2016.06.006
- NATALIA CASTRO ET AL: "Melt Extrusion Encapsulation of Flavors: A Review", POLYMER REVIEWS, vol. 56, no. 1, 2 January 2016 (2016-01-02), US, pages 137 - 186, XP055719414, ISSN: 1558-3724, DOI: 10.1080/15583724.2015.1091776
- ANONYMOUS: "Orange oil - Wikipedia", 23 June 2020 (2020-06-23), pages 1 - 4, XP055719437, Retrieved from the Internet <URL:https://en.wikipedia.org/wiki/Orange_oil> [retrieved on 20200731]
- YULIANI S ET AL: "Extrusion of mixtures of starch and d-limonene encapsulated with @b-cyclodextrin: Flavour retention and physical properties", FOOD RESEARCH INTERNATIONAL, ELSEVIER, AMSTERDAM, NL, vol. 39, no. 3, 1 April 2006 (2006-04-01), pages 318 - 331, XP025014222, ISSN: 0963-9969, [retrieved on 20060401], DOI: 10.1016/J.FOODRES.2005.08.005
- REPKA MICHAEL A ET AL: "Melt extrusion with poorly soluble drugs - An integrated review", INTERNATIONAL JOURNAL OF PHARMACEUTICS, ELSEVIER, NL, vol. 535, no. 1, 2 November 2017 (2017-11-02), pages 68 - 85, XP085328532, ISSN: 0378-5173, DOI: 10.1016/J.IJPHARM.2017.10.056
- TACKENBERG MARKUS W ET AL: "Encapsulation of orange terpenes investigating a plasticisation extrusion process.", JOURNAL OF MICROENCAPSULATION 2015, vol. 32, no. 4, 2015, pages 408 - 417, XP009522045, ISSN: 1464-5246
- RYAN C. BENNETT ET AL: "Preparation of amorphous solid dispersions by rotary evaporation and KinetiSol Dispersing: approaches to enhance solubility of a poorly water-soluble gum extract", JOURNAL DRUG DEVELOPMENT AND INDUSTRIAL PHARMACY, vol. 41, no. 3, 4 March 2015 (2015-03-04), US, pages 382 - 397, XP055719383, ISSN: 0363-9045, DOI: 10.3109/03639045.2013.866142

## Description

La présente invention se rapporte à une composition comprenant au moins un triterpène et/ou au moins un triterpénoïde et/ou au moins une de leurs formes glycosylées, à son procédé de fabrication et à son utilisation.

Les triterpènes et les triterpénoïdes sont largement distribués dans le règne végétal et présentent des activités biologiques variées. Les termes « triterpènes » et « triterpénoïdes » désignent des substances d'origine organique en C30 (30 atomes de carbone) de la famille des terpènes. Généralement, le terme « triterpène » est utilisé pour décrire des triterpènes naturels et le terme plus large « triterpénoïde » désigne à la fois les triterpènes naturels et leurs dérivés fonctionnalisés et/ou issus de la métabolisation qui maintiennent la structure triterpénique. La classification dans l'une ou l'autre des catégories des triterpènes et des triterpénoïdes n'est pas toujours claire dans la littérature et varie selon les auteurs. C'est pourquoi les termes « triterpènes » et « triterpénoïdes » sont fréquemment utilisés pour décrire et désigner les mêmes composés terpéniques en C30 (Parmar et al., Neuropharmacological effects of triterpenoids. Phytopharmacology 2013, 4(2), 354-372). Par exemple, certaines classifications considèrent les acides boswelliques comme étant des triterpénoïdes pentacycliques alors que d'autres classifications considèrent ces mêmes molécules comme étant des triterpènes pentacycliques.

Deux grandes catégories sont à distinguer : les triterpènes ou triterpénoïdes tétracycliques et les triterpènes ou triterpénoïdes pentacycliques. Parmi les triterpènes ou triterpénoïdes tétracycliques, on retrouve notamment l'oléandrine, l'euphol et les cucurbitacines. Dans la catégorie des triterpènes ou triterpénoïdes pentacycliques, on retrouve notamment l'acide bétulinique, l'acide oléanolique, les acides boswelliques, l'acide ursolique, le lupéol, l'acide asiatique et l'acide maslinique.

Parmi les triterpènes ou triterpénoïdes pentacycliques, on distingue les groupes de composés suivants : hopane, arborane, fernane, gammacérane, onocérane, serratane, stictane, oléanane, ursane, taraxastane et lupane.

Les triterpènes ou triterpénoïdes tétracycliques et les triterpènes ou triterpénoïdes pentacycliques constituent des groupes de produits naturels des plus intéressants en raison de leurs diverses activités pharmacologiques : anticancereux, anti-inflammatoire, analgesique, antimicrobien (antibactérien, antiviral, antifongique, antiplasmodique, ...), hépatoprotecteur et cardioprotecteur (antihypertenseur, antidiabétique, antihyperlipidémique, antioxidant, ...), neuroprotecteur (antidépresseur, antifatigue, actions contre des désordres neurologiques tels que Parkinson, Alzheimer, ...).

En particulier et de façon non limitative, les acides boswelliques sont des anti-inflammatoires puissants, des inhibiteurs de la production de médiateurs de l'inflammation, des inhibiteurs de l'activation du NF-kappaB, diminuent les interleukines IL-1, IL-2, IL-4, IL-6 et l'interféron-gamma ; agissent sur la production d'anticorps et l'immunité à médiation cellulaire ; inhibent la 5-lipoxygénase (5-LOX) impliquée dans la biosynthèse des leucotriènes en agissant directement sur un site enzymatique par l'intermédiaire de la structure triterpène ou triterpénoïde pentacyclique et induisent l'apoptose de certaines cellules cancéreuses en inhibant la topoisomérase. Les propriétés anti-inflammatoires des acides boswelliques sont notamment exploitées pour le traitement de plusieurs maladies où l'inflammation est impliquée, comme l'arthrose, l'arthrite, la polyarthrite rhumatoïde, l'asthme, le psoriasis ou encore les maladies inflammatoires chroniques de l'intestin.

Notons qu'il existe de nombreux acides α- et β-boswelliques comme par exemple l'acide α-boswellique, l'acide acétyl-α-boswellique, l'acide β-boswellique, l'acide acétyl-β-boswellique, l'acide 9,11-dehydro-α-boswellique, l'acide acétyl-9,11-dehydro-α-boswellique, l'acide 9,11-dehydro-β-boswellique, l'acide acétyl-9,11-dehydro-β-boswellique, l'acide 11-céto-β-boswellique, l'acide 11-céto- α-boswellique, l'acide 3-acétyl-11-céto-α-boswellique ou l'acide 3-acétyl-11-céto-β-boswellique.

Il est largement reconnu que les triterpènes, les triterpénoïdes et que leurs formes glycosylées, en particulier que les triterpènes ou triterpénoïdes tétracycliques et que les triterpènes ou triterpénoïdes pentacycliques, sont des composés très peu voire totalement insolubles dans l'eau dans laquelle ils ne se dispersent d'ailleurs que faiblement ou pas du tout, ces composés présentant dès lors de très faibles biodisponibilités. Pourtant, malgré leur faible biodisponibilité/bioaccessibilité (c'est-à-dire malgré la faible fraction de la dose administrée qui atteint effectivement la circulation sanguine sous forme inchangée) mais aussi malgré leur faible solubilité et/ou malgré leur faible dispersion notamment dans le milieu intestinal, les effets positifs des triterpènes et des triterpénoïdes et de leurs formes glycosylées, en particulier des triterpènes ou triterpénoïdes tétracycliques et des triterpènes ou triterpénoïdes pentacycliques, sur diverses pathologies en font des molécules d'intérêt en vue d'une administration à l'être humain et/ou pour un usage vétérinaire.

C'est pourquoi, de nombreuses méthodologies et de nombreux procédés ont été développés afin de formuler ces composés sous forme de particules sphériques, de flakes, de pellets ou encore de granulés. En particulier, des techniques d'extrusion peuvent être employées comme par exemple la technique d'extrusion-sphéronisation ou la technique de granulation par extrusion (TSG ou Tween Screw Granulation).

La technique d'extrusion-sphéronisation, telle que décrite par exemple dans le document EP1391426, permet de produire des particules rondes de taille homogène au départ d'une masse humide (contenant une substance active et au moins un excipient) qui est passée au travers d'une grille présentant un maillage prédéterminé avant séchage des particules ainsi obtenues. Plus particulièrement, ce procédé de mise en forme de poudre repose sur les étapes suivantes : mélange d'une substance active et d'au moins un excipient, granulation (compaction) humide du mélange précédemment obtenu, extrusion du mélange compacté pour obtenir un extrudat, sphéronisation de l'extrudat pour former des particules/granulés sphériques et séchage des particules/granulés sphériques obtenus.

La technique de granulation par extrusion, telle que décrite par exemple dans le document US5260074, permet quant à elle d'obtenir des produits intermédiaires pour la préparation de comprimés et de gélules. Cette technique repose sur une granulation (compaction) de substances sous forme de poudres dans une extrudeuse pour donner lieu à la formation de granulés en sortie de ce dernier.

Malheureusement, il apparait que les formulations actuelles comprenant des triterpènes et/ou des triterpénoïdes et/ou de leurs formes glycosylées, en particulier des triterpènes ou triterpénoïdes tétracycliques et/ou des triterpènes ou triterpénoïdes pentacycliques, sont peu appropriées en raison de leur trop faible voire de leur non solubilité et/ou en raison de leur trop faible voire de leur non dispersion en phase aqueuse et/ou en raison du faible relargage de ces composés au départ de ces formulations, ce qui se traduit finalement par une faible biodisponibilité/bioaccessibilité de ces molécules d'intérêt. Notons qu'il apparait aussi que les procédés actuels de fabrication de ces formulations sont contraignants et difficiles à mettre en œuvre.

RYAN C. BENNETT ET AL (Journal Drug Development and Industrial Pharmacy, vol. 41, no. 3, 2015-03-04, p. 382-397) décrit la préparation d'une solide dispersion amorphe d'acides boswelliques en utilisant la technique Kinetisol. Gao Nannan ET AL (Asian Journal of Pharmaceutical Sciences, vol. 12, no. 1, 2017-01-01, p. 66-72) a trait à l'amélioration de la dissolution de l'acide oléanique via une dispersion solide amorphe obtenue par extrusion à chaud avec des polymères à base de PVP ou PEG.

Par les termes « dispersion en phase aqueuse (en milieu aqueux) », il est entendu, au sens de la présente invention, un système composé de deux phases dans lequel l'une des deux phases, appelée phase dispersée, est finement divisée dans l'autre, appelée phase dispersante. Cette dispersion peut être moléculaire (solution), colloïdale (dispersion de particules submicroniques) ou plus grossière (dispersion de particules supérieures au µm). Plus particulièrement, selon l'invention, les termes « dispersion en phase aqueuse » désigne les suspensions, constituées d'une phase solide dispersée dans une phase aqueuse (liquide).

Au sens de la présente invention, le terme « solubilité », désigne la capacité d'une substance, appelée soluté, à se dissoudre dans une autre substance, appelée solvant, pour former un mélange homogène appelé solution.

L'invention a pour but de pallier au moins en partie les inconvénients de l'état de la technique en procurant (1) une composition comprenant au moins un triterpène et/ou au moins un triterpénoïde et/ou au moins une de leurs formes glycosylées dont la (les) solubilité(s) et/ou la (les) dispersion(s) en phase aqueuse (milieu aqueux) est (sont) augmentée(s) de telle sorte que la (les) biodisponibilité(s) de ces composés soit (soient) significativement augmentée(s) et (2) un procédé de fabrication d'une telle composition qui soit facile à mettre en œuvre, qui soit flexible, qui soit économiquement rentable et qui assure que ledit au moins un triterpène et/ou ledit au moins un triterpénoïde et/ou ladite au moins une de leurs formes glycosylées soit (soient) présent(s) et réparti(s) de façon homogène dans la composition finale obtenue.

Par ailleurs, l'invention entend procurer une composition qui est stable au cours du temps, c'est-à-dire qui conserve ses propriétés en termes de solubilité et/ou de dispersion dudit au moins un triterpène et/ou dudit au moins un triterpénoïde et/ou de ladite au moins une de leurs formes glycosylées et qui conserve ses propriétés en termes de taux de relargage de ces composés au cours du temps au départ d'une composition (formulation) selon l'invention, ceci en particulier en phase aqueuse et plus particulièrement dans le milieu intestinal.

Pour résoudre au moins partiellement ces problèmes, il est prévu suivant l'invention, une composition sous forme d'un extrudat thermoformé, éventuellement conditionné en pellet, en flake, en granulé, en poudre, en comprimé effervescent ou non, en solution injectable ou non, en suspension, en gel, en pommade ou encore sous toute autre forme adéquate permettant une administration à un animal ou à un être humain, comprenant au moins un triterpène et/ou au moins un triterpénoïde et/ou au moins une de leurs formes glycosylées et au moins un polymère choisi dans le groupe constitué des protéines naturelles ou synthétiques, des oligosaccharides naturels ou synthétiques, des polysaccharides naturels ou synthétiques, de leurs dérivés et de leurs mélanges, ledit au moins un triterpène et/ou ledit au moins un triterpénoïde et/ou ladite au moins une de leurs formes glycosylées comprenant au moins une première phase amorphe et éventuellement une deuxième phase cristalline.

En particulier, selon l'invention, ledit au moins un polymère est un polymère thermoplastique, c'est-à-dire un polymère ayant la propriété de se ramollir lorsqu'il est chauffé suffisamment, mais qui, se refroidissant, redevient dur.

Selon un mode de réalisation, il est donc prévu suivant l'invention, une composition sous forme d'un extrudat thermoformé comprenant au moins un triterpène et/ou au moins un triterpénoïde et/ou au moins une de leurs formes glycosylées et au moins une protéine naturelle ou synthétique en tant que polymère, ledit au moins un triterpène et/ou ledit au moins un triterpénoïde et/ou ladite au moins une de leurs formes glycosylées comprenant au moins une première phase amorphe et éventuellement une deuxième phase cristalline.

Selon un mode de réalisation, il est également prévu suivant l'invention, une composition sous forme d'un extrudat thermoformé comprenant au moins un triterpène et/ou au moins un triterpénoïde et/ou au moins une de leurs formes glycosylées et au moins un oligosaccharide naturel ou synthétique en tant que polymère, ledit au moins un triterpène et/ou ledit au moins un triterpénoïde et/ou ladite au moins une de leurs formes glycosylées comprenant au moins une première phase amorphe et éventuellement une deuxième phase cristalline.

Selon un mode de réalisation, il est également prévu suivant l'invention, une composition sous forme d'un extrudat thermoformé comprenant au moins un triterpène et/ou au moins un triterpénoïde et/ou au moins une de leurs formes glycosylées et au moins un polysaccharide naturel ou synthétique en tant que polymère, ledit au moins un triterpène et/ou ledit au moins un triterpénoïde et/ou ladite au moins une de leurs formes glycosylées comprenant au moins une première phase amorphe et éventuellement une deuxième phase cristalline.

Par le terme « extrudat », il est entendu, au sens de la présente invention, une matière qui sort d'une extrudeuse, en particulier de la filière d'une extrudeuse.

Par les termes « extrudat thermoformé », il est entendu, au sens de la présente invention, une matière qui sort d'un appareillage, en particulier qui sort d'une extrudeuse, dans lequel elle a subi une transformation par l'effet de la chaleur, éventuellement par l'effet combiné de la chaleur et de forces de cisaillement d'une vis sans fin. Une telle transformation par l'effet de la chaleur, éventuellement par l'effet combiné de la chaleur et de forces de cisaillement d'une vis sans fin, peut être obtenue avec la technique de l'extrusion à chaud (HME ou Hot Melt Extrusion).

En particulier, un extrudat thermoformé selon l'invention est un extrudat dans lequel le(s) principe(s) actif(s) (ledit au moins un triterpènre et/ou ledit au moins un triterpénoïde et/ou ladite au moins une de leurs formes glycosylées) et/ou ledit au moins un polymère est/sont fondu(s).

En particulier, la composition selon l'invention, plus particulièrement la composition sous forme d'un extrudat thermoformé selon l'invention, est une dispersion solide dans laquelle ledit au moins un triterpène et/ou ledit au moins un triterpénoïde et/ou ladite au moins une de leurs formes glycosylées comprenant au moins une première phase amorphe et éventuellement une deuxième phase cristalline est/sont dispersé(s) dans ledit au moins un polymère choisi dans le groupe constitué des protéines naturelles ou synthétiques, des oligosaccharides naturels ou synthétiques, des polysaccharides naturels ou synthétiques, de leurs dérivés et de leurs mélanges.

Selon un mode de réalisation, il est donc prévu suivant l'invention, une composition sous forme d'un extrudat thermoformé comprenant au moins un triterpène et/ou au moins un triterpénoïde et/ou au moins une de leurs formes glycosylées et au moins une protéine naturelle ou synthétique en tant que polymère, ledit au moins un triterpène et/ou ledit au moins un triterpénoïde et/ou ladite au moins une de leurs formes glycosylées comprenant au moins une première phase amorphe et éventuellement une deuxième phase cristalline, ladite composition étant une dispersion solide dans laquelle ledit au moins un triterpène et/ou ledit au moins un triterpénoïde et/ou ladite au moins une de leurs formes glycosylées est/sont dispersé(s) dans ladite au moins une protéine naturelle ou synthétique en tant que polymère, lequel est/a été fondu.

Selon un mode de réalisation, il est également prévu suivant l'invention, une composition sous forme d'un extrudat thermoformé comprenant au moins un triterpène et/ou au moins un triterpénoïde et/ou au moins une de leurs formes glycosylées et au moins un oligosaccharide naturel ou synthétique en tant que polymère, ledit au moins un triterpène et/ou ledit au moins un triterpénoïde et/ou ladite au moins une de leurs formes glycosylées comprenant au moins une première phase amorphe et éventuellement une deuxième phase cristalline, ladite composition étant une dispersion solide dans laquelle ledit au moins un triterpène et/ou ledit au moins un triterpénoïde et/ou ladite au moins une de leurs formes glycosylées est/sont dispersé(s) dans ledit au moins un oligosaccharide naturel ou synthétique en tant que polymère, lequel est/a été fondu.

Selon un mode de réalisation, il est également prévu suivant l'invention, une composition sous forme d'un extrudat thermoformé comprenant au moins un triterpène et/ou au moins un triterpénoïde et/ou au moins une de leurs formes glycosylées et au moins un polysaccharide naturel ou synthétique en tant que polymère, ledit au moins un triterpène et/ou ledit au moins un triterpénoïde et/ou ladite au moins une de leurs formes glycosylées comprenant au moins une première phase amorphe et éventuellement une deuxième phase cristalline, ladite composition étant une dispersion solide dans laquelle ledit au moins un triterpène et/ou ledit au moins un triterpénoïde et/ou ladite au moins une de leurs formes glycosylées est/sont dispersé(s) dans ledit au moins un polysaccharide naturel ou synthétique en tant que polymère, lequel est/a été fondu.

Un extrudat thermoformé selon l'invention est obtenu par thermoformage à chaud, en particulier par thermoformage à chaud par la technique d'extrusion à chaud. Selon l'invention, le thermoformage à chaud concerne donc plus particulièrement la technique de l'extrusion à chaud.

Il est donc prévu selon l'invention une composition obtenue par thermoformage à chaud sous forme d'un extrudat thermoformé obtenu par thermoformage à chaud, en particulier obtenu par extrusion à chaud, ladite composition comprenant au moins un triterpène et/ou au moins un triterpénoïde et/ou au moins une de leurs formes glycosylées et au moins un polymère choisi dans le groupe constitué des protéines naturelles ou synthétiques, des oligosaccharides naturels ou synthétiques, des polysaccharides naturels ou synthétiques, de leurs dérivés et de leurs mélanges, ledit au moins un triterpène et/ou ledit au moins un triterpénoïde et/ou ladite au moins une de leurs formes glycosylées comprenant au moins une première phase amorphe et éventuellement une deuxième phase cristalline.

Un extrudat thermoformé selon l'invention peut donc être obtenu selon la technique d'extrusion à chaud qui permet de réaliser la dispersion moléculaire d'un actif (substance active) au sein d'une matrice polymérique (au sein d'un polymère) pour former des dispersions solides. Cette dispersion solide est possible grâce à un apport de chaleur et éventuellement grâce à la contrainte appliquée par le mouvement des vis sans fin sur la matière dans un extrudeur. En final, l'extrusion à chaud donne lieu, en sortie, à la formation d'un extrudat thermoformé sous forme d'un jonc qui peut alors notamment être pelletisé ou broyé.

Si la technique d'extrusion-sphéronisation et la technique de granulation par extrusion telles que décrites plus haut sont réalisées sans apport de chaleur (chauffage) et qu'elles requièrent typiquement une phase liquide (généralement de l'eau) pour obtenir des particules sphériques et/ou des granulés, la technique d'extrusion à chaud peut être réalisée sans apport de cette phase liquide mais repose sur un apport de chaleur (chauffage) pour assurer une transformation de la matière par thermoformage.

Par ailleurs, si la technique d'extrusion-sphéronisation et la technique de granulation par extrusion consistent en une agglomération en granules de poudres en essayant autant que possible de conserver les propriétés initiales des constituants de ces poudres, la technique d'extrusion à chaud donne lieu au contraire à une transformation de la matière, en particulier à une structure vitreuse (« glassy structure ») obtenue sous l'action de la chaleur (chauffage), les particules constituant les poudres n'étant plus toutes présentes sous leur forme initiale (native) cristalline en fin de procédé d'extrusion à chaud mais ayant subis une transformation par thermoformage.

Par les termes « comprenant au moins une première phase amorphe et éventuellement une deuxième phase cristalline », il est entendu, au sens de la présente invention, que ledit au moins un triterpène et/ou ledit au moins un triterpénoïde et/ou ladite au moins une de leurs formes glycosylées peut (peuvent) soit comprendre 100% en masse d'une phase amorphe, soit qu'il(s) peut (peuvent) comprendre simultanément une première phase amorphe et une deuxième phase cristalline, la somme des pourcentages en masse des première et deuxième phases étant dans ce cas égale à 100. En d'autres termes, la composition selon l'invention peut comprendre ledit au moins un triterpène et/ou ledit au moins un triterpénoïde et/ou ladite au moins une de leurs formes glycosylées (1) totalement sous forme amorphe ou (2) partiellement sous forme (phase) amorphe et partiellement sous forme (phase) cristalline.

Notons qu'une phase est dite amorphe lorsque les atomes la constituant ne respectent aucun ordre à moyenne et grande distance, ce qui la distingue d'une phase dite cristalline.

La composition selon l'invention se présente donc sous forme d'un extrudat thermoformé dans lequel ledit au moins un triterpène et/ou ledit au moins un triterpénoïde et/ou ladite au moins une de leurs formes glycosylées (principe actif) comprend (comprennent) au moins une première phase amorphe et éventuellement une deuxième phase cristalline, laquelle ou lesquelles phases(s) est/sont dispersée(s) au sein d'au moins un polymère choisi dans le groupe constitué des protéines naturelles ou synthétiques, des oligosaccharides naturels ou synthétiques, des polysaccharides naturels ou synthétiques, de leurs dérivés et de leurs mélanges.

Par les termes « protéine naturelle », il est entendu au sens de la présente invention toute protéine naturellement présente dans le monde du vivant, en particulier une protéine végétale ou animale.

Par les termes « protéine synthétique », il est entendu au sens de la présente invention toute protéine faisant l'objet d'une intervention humaine, en particulier une protéine obtenue au départ d'un procédé chimique ou biochimique ou biotechnologique.

Par les termes « oligosaccharides naturels » et « polysaccharides naturels », il est entendu au sens de la présente invention tout oligosaccharide et tout polysaccharide naturellement présent dans le monde du vivant, en particulier un oligosaccharide ou un polysaccharide végétal ou animal.

Par les termes « oligosaccharides synthétiques » et « polysaccharides synthétiques », il est entendu au sens de la présente invention tout oligosaccharide et tout polysaccharide faisant l'objet d'une intervention humaine, en particulier tout oligosaccharide et tout polysaccharide obtenu au départ d'un procédé chimique ou biochimique ou biotechnologique.

Il a été déterminé, dans le cadre de la présente invention, qu'une telle composition sous forme d'un extrudat thermoformé comprenant au moins un triterpène et/ou au moins un triterpénoïde et/ou au moins une de leurs formes glycosylées comme principe(s) actif(s) et au moins un polymère choisi dans le groupe constitué des protéines naturelles ou synthétiques, des oligosaccharides naturels ou synthétiques, des polysaccharides naturels ou synthétiques, de leurs dérivés et de leurs mélanges et dans laquelle ledit au moins un triterpène et/ou ledit au moins un triterpénoïde et/ou ladite au moins une de leurs formes glycosylées comprend (comprennent) au moins une première phase amorphe et éventuellement une deuxième phase cristalline présente une (des) solubilité(s) et/ou une (des) dispersion(s) en phase aqueuse (milieu aqueux) nettement supérieure(s) dudit au moins un triterpène et/ou dudit au moins un triterpénoïde et/ou de ladite au moins une de leurs formes glycosylées. Par ailleurs, il a été montré qu'une telle composition selon l'invention présente une (des) biodisponibilité(s)/ bioaccessibilité(s)significativement augmentée(s) dudit au moins un triterpène et/ou dudit au moins un triterpénoïde et/ou de ladite au moins une de leurs formes glycosylées par rapport aux biodisponibilités/bioaccessibilités observées pour les compositions actuelles.

Selon l'invention, le(s) principe(s) actif(s), c'est-à-dire ledit au moins un triterpène et/ou ledit au moins un triterpénoïde et/ou ladite au moins une de leurs formes glycosylées comprenant au moins une première phase amorphe et éventuellement une deuxième phase cristalline, est (sont) dispersés/répartis/distribués de manière homogène au sein d'au moins un polymère choisi dans le groupe constitué des protéines naturelles ou synthétiques, des oligosaccharides naturels ou synthétiques, des polysaccharides naturels ou synthétiques, de leurs dérivés et de leurs mélanges, le(s) principe(s) actif(s) et/ou ledit au moins un polymère étant fondu lors du procédé de fabrication par thermoformage mis en œuvre selon l'invention et décrit plus loin.

En outre, une composition selon l'invention peut être conservée plusieurs mois sans que ses propriétés ne soient altérées. En particulier, il a été mis en évidence qu'une composition selon l'invention conserve ses propriétés en termes de solubilité(s) et/ou de dispersion(s) en phase aqueuse (milieu aqueux) dudit au moins un triterpène et/ou dudit au moins un triterpénoïde et/ou de ladite au moins une de leurs formes glycosylées et en termes de taux de relargage de ce(s) composé(s) au cours du temps au départ d'une composition/formulation selon l'invention, ceci en particulier en phase aqueuse.

Avantageusement, selon l'invention, ledit extrudat thermoformé comprend un mélange thermoformé dudit au moins un triterpène et/ou dudit au moins un triterpénoïde et/ou de ladite au moins une de leurs formes glycosylées et dudit au moins un polymère choisi dans le groupe constitué des protéines naturelles ou synthétiques, des oligosaccharides naturels ou synthétiques, des polysaccharides naturels ou synthétiques, de leurs dérivés et de leurs mélanges.

Alternativement, selon l'invention, ledit extrudat thermoformé est constitué d'un mélange thermoformé d'au moins un triterpène et/ou d'au moins un triterpénoïde et/ou d'au moins une de leurs formes glycosylées et d'au moins un polymère choisi dans le groupe constitué des protéines naturelles ou synthétiques, des oligosaccharides naturels ou synthétiques, des polysaccharides naturels ou synthétiques, de leurs dérivés et de leurs mélanges.

Il est donc prévu selon l'invention un extrudat thermoformé obtenu par thermoformage à chaud, en particulier obtenu par extrusion à chaud, ledit extrudat thermoformé comprenant un mélange thermoformé d'au moins un triterpène et/ou d'au moins un triterpénoïde et/ou d'au moins une de leurs formes glycosylées et d'au moins un polymère choisi dans le groupe constitué des protéines naturelles ou synthétiques, des oligosaccharides naturels ou synthétiques, des polysaccharides naturels ou synthétiques, de leurs dérivés et de leurs mélanges.

Par les termes « mélange thermoformé », il est entendu, au sens de la présente invention, un mélange qui sort d'un appareillage, en particulier qui sort d'une extrudeuse, dans lequel il a subi une transformation par l'effet de la chaleur, éventuellement par l'effet combiné de la chaleur et de forces de cisaillement d'une vis sans fin. Une telle transformation par l'effet de la chaleur, éventuellement par l'effet combiné de la chaleur et de forces de cisaillement d'une vis sans fin, peut être obtenue avec la technique de l'extrusion à chaud (HME).

En particulier, un mélange thermoformé selon l'invention est un mélange dans lequel le(s) principe(s) actif(s) (ledit au moins un triterpènre et/ou ledit au moins un triterpénoïde et/ou ladite au moins une de leurs formes glycosylées) et/ou ledit au moins un polymère est/sont fondu(s) / a été/ont été fondu(s).

Selon l'invention, ledit au moins un triterpène et/ou ledit au moins un triterpenoide et/ou ladite au moins une de leurs formes glycosylées comprend (comprennent) une première phase amorphe.

Avantageusement, selon l'invention, ledit au moins un triterpène et/ou ledit au moins un triterpénoïde et/ou ladite au moins une de leurs formes glycosylées comprend (comprennent) majoritairement au moins une première phase amorphe.

De préférence, selon l'invention, ledit au moins un triterpène et/ou ledit au moins un triterpénoïde et/ou ladite au moins une de leurs formes glycosylées comprend entre 51 et 100% en masse d'une phase amorphe et entre 0 et 49% en masse d'une phase cristalline.

Par les termes « majoritairement au moins une première phase amorphe », il est donc entendu, au sens de la présente invention, que ledit au moins un triterpène et/ou ledit au moins un triterpénoïde et/ou ladite au moins une de leurs formes glycosylées comprend (comprennent) entre 50 et 100% en masse d'une phase amorphe et entre 0 et 50% d'une phase cristalline, plus particulièrement que ledit au moins un triterpène et/ou ledit au moins un triterpénoïde et/ou ladite au moins une de leurs formes glycosylées comprend (comprennent) entre 51 et 100% en masse d'une phase amorphe et entre 0 et 49% d'une phase cristalline.

Avantageusement, selon l'invention, ledit au moins un triterpène et/ou ledit au moins un triterpénoïde et/ou ladite au moins une de leurs formes glycosylées est tétracyclique, comme par exemple l'oléandrine, l'euphol ou la cucurbitacine, ou pentacyclique, comme par exemple l'acide bétulinique, l'acide oléanolique, un acide boswellique, l'acide ursolique, le lupéol, l'acide asiatique, l'acide madécassique, l'acide maslinique, la jujubogenine ou la pseudojujubogenine.

Les formes glycosylées des triterpènes, comme par exemple les ginsenosides, tombent également sous le couvert de la présente invention. Les formes glycosylées des triterpénoïdes, comme par exemple les bacosides, les asiaticosides et l'α-héderine tombent également sous le couvert de la présente invention.

Avantageusement, selon l'invention, ledit acide boswellique est choisi dans le groupe constitué des acides α- et β-boswelliques, par exemple l'acide α-boswellique, l'acide acétyl-α-boswellique, l'acide β-boswellique, l'acide acétyl-β-boswellique, l'acide 9,11-dehydro-α-boswellique, l'acide acétyl-9,11-dehydro-α-boswellique, l'acide 9,11-dehydro-β-boswellique, l'acide acétyl-9,11-dehydro-β-boswellique, l'acide 11-céto-β-boswellique, l'acide 11-céto- α-boswellique, l'acide 3-acétyl-11-céto-α-boswellique, l'acide 3-acétyl-11-céto-β-boswellique.

Préférentiellement, selon l'invention, lesdites protéine naturelles ou synthétiques sont choisies dans le groupe constitué des glycoprotéines, des collagènes et/ou des hydrolysats de collagène, des protéines végétales, des protéines animales, de leurs dérivés et de leurs mélanges.

Par les termes « hydrolysat de collagène », il est entendu, au sens de la présente invention, les gélatines et les collagènes hydrolysés ou peptides de collagène. Les termes « hydrolysat de collagène » englobent donc les gélatines ayant la capacité de gélifier mais aussi les collagènes hydrolysés ou peptides de collagène qui présentent ou non la capacité de gélifier.

A titre d'exemple, lorsque ladite au moins une protéine naturelle ou synthétique est du collagène ou de la gélatine, il peut s'agir de collagène ou de gélatine d'origine animale (poisson, porc, bœuf, ...).

A titre d'exemple, lorsque ladite au moins une protéine naturelle ou synthétique est une protéine végétale, il peut s'agir d'une protéine de soja, de citrouille, de riz, de blé, de petit pois ou de noix. Cette liste est non exhaustive.

Préférentiellement, selon l'invention, lesdits collagènes et/ou lesdits hydrolysats de collagène présentent un poids moléculaire compris entre 50 et 300000 Da, de préférence entre 100 et 275000 Da, de préférence entre 150 et 250000 Da, de préférence entre 200 et 225000 Da, de préférence entre 250 et 200000 Da, de préférence entre 300 et 175000 Da, de préférence entre 350 et 150000 Da, de préférence entre 400 et 125000 Da, de préférence entre 450 et 100000 Da, de préférence entre 500 et 75000 Da, de préférence entre 550 et 50000 Da, de préférence entre 600 et 40000 Da, de préférence entre 650 et 30000 Da, de préférence entre 700 et 20000 Da, de préférence entre 750 et 10000 Da, de préférence entre 800 et 9000 Da, de préférence entre 850 et 8000 Da, de préférence entre 900 et 7000 Da, de préférence entre 950 et 6000 Da, de préférence entre 1000 et 5000 Da, de préférence entre 1050 et 4000 Da, de préférence entre 1100 et 3000 Da, de préférence entre 1150 et 2000 Da, de préférence entre 1200 et 1000 Da.

Avantageusement, selon l'invention, lesdits collagènes et/ou lesdits hydrolysats de collagène présentent un poids moléculaire compris entre 1000 et 300000 Da, de préférence entre 1500 et 150000 Da, de préférence entre 2000 et 60000 Da.

De préférence, selon l'invention, lesdits collagènes et/ou lesdits hydrolysats de collagène présentent un poids moléculaire égal à 50 Da ou égal à 100 Da ou égal à 150 Da ou égal à 200 Da ou égal à 250 Da ou égal à 300 Da ou égal à 350 Da ou égal à 400 Da ou égal à 450 Da ou égal à 500 Da ou égal à 550 Da ou égal à 600 Da ou égal à 650 Da ou égal à 700 Da ou égal à 750 Da ou égal à 800 Da ou égal à 850 Da ou égal à 900 ou égal à 950 Da ou égal à 1000 Da ou égal à 1100 Da ou égal à 1200 ou égal à 1300 Da ou égal à 1400 Da ou égal à 1500 Da ou égal à 1600 Da ou égal à 1700 Da ou égal à 1800 Da ou égal à 1900 Da ou égal à 2000 Da ou égal à 2500 Da ou égal à 3000 Da ou égal à 3500 Da ou égal à 4000 Da ou égal à 4500 Da ou égal à 5000 Da ou égal à 5500 Da ou égal à 6000 Da ou égal à 6500 Da ou égal à 7000 Da ou égal à 7500 Da ou égal à 8000 Da ou égal à 8500 Da ou égal à 9000 Da ou égal à 9500 Da ou égal à 10000 Da ou égal à 12500 Da ou égal à 15000 Da ou égal à 17500 Da ou égal à 20000 Da ou égal à 22500 Da ou égal à 25000 Da ou égal à 27500 Da ou égal à 30000 Da ou égal à 32500 Da ou égal à 35000 Da ou égal à 37500 Da ou égal à 40000 Da ou égal à 42500 Da ou égal à 45000 Da ou égal à 47500 Da ou égal à 50000 Da ou égal à 55000 Da ou égal à 60000 Da ou égal à 65000 Da ou égal à 70000 Da ou égal à 75000 Da ou égal à 80000 Da ou égal à 85000 Da ou égal à 90000 Da ou égal à 100000 Da ou égal à 110000 Da ou égal à 120000 Da ou égal à 130000 Da ou égal à 140000 Da ou égal à 150000 Da ou égal à 160000 Da ou égal à 170000 Da ou égal à 180000 Da ou égal à 190000 da ou égal à 200000 Da ou égal à 210000 Da ou égal à 220000 Da ou égal à 230000 Da ou égal à 240000 Da ou égal à 250000 Da ou égal à 260000 Da ou égal à 270000 Da ou égal à 280000 Da ou égal à 290000 Da ou égal à 300000 Da.

Suivant un mode de réalisation selon l'invention, lorsque ladite au moins une protéine naturelle ou synthétique est du collagène, ce dernier présente un poids moléculaire compris entre 900 et 7000 Da, de préférence un poids moléculaire compris entre 950 et 5000 Da, préférentiellement un poids moléculaire compris entre 1000 et 3000 Da.

Suivant un mode de réalisation selon l'invention, lorsque ladite au moins une protéine naturelle ou synthétique est du collagène, ce dernier présente un poids moléculaire égal à 2000 Da ou égal à 3000 Da ou égal à 5000 Da ou égal à 50000 Da.

Suivant un mode de réalisation selon l'invention, lorsque ladite au moins une protéine naturelle ou synthétique est de la gélatine, cette dernière présente un poids moléculaire compris entre 900 et 6000 Da, de préférence un poids moléculaire compris entre 950 et 5000 Da, préférentiellement un poids moléculaire compris entre 1000 et 3000 Da.

Suivant un mode de réalisation selon l'invention, lorsque ladite au moins une protéine naturelle ou synthétique est de la gélatine, cette dernière présente un poids moléculaire égal à 2000 Da ou égal à 3000 Da ou égal à 5000 Da ou égal à 50000 Da.

Suivant un mode de réalisation selon l'invention, lorsque ladite au moins une protéine naturelle ou synthétique est un collagène hydrolysé ou un peptide de collagène, ce dernier présente un poids moléculaire compris entre 900 et 6000 Da, de préférence un poids moléculaire compris entre 950 et 5000 Da, préférentiellement un poids moléculaire compris entre 1000 et 3000 Da.

Suivant un mode de réalisation selon l'invention, lorsque ladite au moins une protéine naturelle ou synthétique est un collagène hydrolysé ou un peptide de collagène, ce dernier présente un poids moléculaire égal à 2000 Da ou égal à 3000 Da ou égal à 5000 Da ou égal à 50000 Da.

De préférence, selon l'invention, lesdits oligosaccharides sont choisis dans le groupe constitué de la cyclodextrine, du raffinose, du rhamminose, du rhamnose, du stachyose, du verbascose, du tréhalose, du lactose, du lactulose, du maltose, de leurs dérivés et de leurs mélanges.

Avantageusement, selon l'invention, lesdits polysaccharides naturels ou synthétiques sont choisis dans le groupe constitué des amidons (amidon de maïs, amidon de pomme de terre, amidon prégélatinisé, ...), des fibres (acacia, inuline, les alginates, les carraghénanes, pectine, ...), des celluloses et des hémicelluloses (par exemple l'hydroxypropylcellulose (HPC), le phtalate d'hydroxypropylméthylcellulose, l'hydroxypropyméthylcellulose (HPMC), l'acétosuccinate d'hydroxypropyméthylcellulose, l'acétobutyrate de cellulose, l'acétophthalate de cellulose), du glycogène, du β-glucane, de l'inuline, de l'amylopectine, de l'amylose, de la dextrine, de la maltodextrine, de l'isomaltose, du xylane, du pullulane, de l'agar-agar, des carraghénanes, des mannanes, du fucoïdane, des gommes (xanthane, guar, mastic ou gomme arabique, ...), du chitosan, de la chitine, du xanthane, de la lévane, de la néoserine, de l'acide hyaluronique, des hyaluronates, du sulfate de chondroïtine, du dermatane sulfate, du kératane sulfate, de leurs dérivés et de leurs mélanges.

Selon un mode de réalisation, la composition suivant l'invention comprend en outre au moins un polymère naturel ou synthétique additionnel choisi dans le groupe constitué de l'acétate de polyvinyle, du polyvinylpyrrolidone (PVP), de l'acétate de polyvinylpyrrolidone-co-vinyle, de l'acétate de polyethylène-co-vinyle, de l'acétate co-méthacrylique d'acide polyvinyle, du polyethylène oxide, du polylactide-co-glycolide, de l'alcool polyvinylique, du polycarbophile, de la polycaprolactone, de la cire de carnauba, du copolymère d'éthylène-vinyle, de la lécithine, de l'huile de ricin, de l'huile de soja hydrogénée, des cires, de l'isomalt, de leurs dérivés et de leurs mélanges.

Avantageusement, la composition selon l'invention comprend en outre au moins un agent plastifiant. L'addition d'un agent plastifiant dans une composition selon l'invention permet d'obtenir une composition selon l'invention au travers d'un procédé de fabrication où des températures inférieures aux points de fusion dudit au moins un triterpène et/ou dudit au moins un triterpénoïde et/ou de ladite au moins une de leurs formes glycosylées et du polymère peuvent être utilisées afin de garantir tout de même une fonte de ces deux composés et la dispersion/répartition/distribution dudit au moins un triterpène et/ou dudit au moins un triterpénoïde et/ou de ladite au moins une de leurs formes glycosylées au sein du polymère.

Préférentiellement, selon l'invention, ledit agent plastifiant est choisi dans le groupe constitué des polyols (glycérol, sorbitol, mannitol, ...), des lipides (acides gras, esters d'acides gras, amides d'acides gras, glycérides, phospholipides, ...), , des esters de sucrose, de l'eau, du citrate de triéthyle, du polyéthylène glycol, , du sébate de dibutyle, du stéarate de butyle, du monostéarate de glycérol, du phtalate diéthyle, de leurs dérivés et de leurs mélanges.

Par exemple, selon l'invention, les phospholipides peuvent être des lécithines comme la lécithine de soja, la lécithine de tournesol ou la lécithine de jaune d'œuf.

Selon l'invention, les agents plastifiants préférés sont le glycérol, l'eau, le polyéthylène glycol et le citrate de triéthyle.

De préférence, la composition selon l'invention comprend en outre au moins un additif choisi dans le groupe constitué des agents lubrifiants, des agents surfactants, des agents antioxydants, des agents chélatants, de leurs dérivés et de leurs mélanges.

A titre d'exemple, peuvent être utilisés, seuls ou en mélange, en tant qu'agents lubrifiants dans une composition selon l'invention les composés suivants : le dibéhénate de glycérol, le talc, la silice, l'acide stéarique, l'acide borique, les cires, l'oléate de sodium, l'acétate de sodium, le stéarate de magnésium, le stéarate de calcium, le stéarate de sodium, le benzoate de sodium, le laurylsulfate de sodium, le distéarate de glycérol, le palmitostéarate de glycérol, la cellulose microcristalline ou encore les polyoxyl-8-glycérides.

A titre d'exemple, peuvent être utilisés, seuls ou en mélange, en tant qu'agents surfactants dans une composition selon l'invention les composés suivants : le Pluronic^{®}, le Span^{®}, le Cremophor^{®}, les polysorbates (Tween^{®}, ...), la vitamine E TPGS et le ducosate de sodium.

A titre d'exemple, peuvent être utilisés, seuls ou en mélange, en tant qu'agents antioxydants et/ou agents chélatants dans une composition selon l'invention les composés suivants : l'hydroxytoluène butylé, l'hydroxyanisiole butylé, l'EDTA, l'acide citrique et la vitamine E.

Avantageusement, la composition selon l'invention comprend en outre au moins un composé additionnel de type polyphénol choisi dans le groupe constitué des acides phénoliques, des stilbènes, des alcools phénoliques, des lignanes, des flavonoïdes, de leurs dérivés et de leurs mélanges. En particulier, les formes glycosylée et aglycone des polyphénols sont envisagées en tant que principe actif additionnel selon la présente invention. Plus particulièrement, au sens de la présente invention, le terme « polyphénol » désigne tant les polyphénols d'origine naturelle que les polyphénols de synthèse mais aussi tous les dérivés de polyphénols.

A titre d'exemple, au sens de la présente invention, peuvent être cités en tant qu'acides phénoliques, les dérivés de l'acide hydroxybenzoïque (acide gallique, acide tannique, ...) et les dérivés de l'acide hydroxycinamique (curcumine, acide coumarique, acide caféique, acide férulique, ...).

A titre d'exemple, au sens de la présente invention, peuvent être cités en tant que stilbènes, le resvératrol, le sirtinol, le picéatannol ou encore la polydatine.

A titre d'exemple, au sens de la présente invention, peuvent être cités en tant que flavonoïdes, les flavanoles (quercétine, myricétine, kaempférol, isorhamnétine, morine, rutine, tiliroside, trihydroxyéthylrutine, fisétine, ...), les flavones (apigénine, lutéoline, baicaléine, chrysine, diosmine, nobilétine, tangérétine, wogonine, aminogénistéine, ...), les flavanones (bavachine, 8-isopenténylnaringénine, isoxanthohumole, naringénine, eriodictyole, hespérétine, silybine, taxifoline, ...), les isoflavones (génistéine, daidzéine, daidzine, formonétine, génistine, néobavaisoflavone, puéranine, ...), les antocianidines (cianidine, pelargonidine, delphinidine, pétunidine, malvidine, ...) et les flavanols (cathéchines, gallocatéchine, épigallocatéchinegallate, ...).

Selon l'invention, ledit au moins un principe actif additionnel de type polyphénol constitue un inhibiteur/modulateur des pompes à efflux dont la P-gp.

Préférentiellement, la composition selon l'invention comprend en outre au moins un inhibiteur et/ou un modulateur de l'activité de la P-gp.

De préférence, la composition selon l'invention est conditionnée sous forme de pellets, de flakes, de granulés, de poudres, de comprimés effervescents ou non, de solutions injectables ou non, de suspensions, de gels, de pommades ou encore sous toute autre forme adéquate permettant une administration à un animal ou à un être humain.

D'autres formes de réalisation d'une composition suivant l'invention sont indiquées dans les revendications annexées.

L'invention a aussi pour objet un procédé de fabrication, en particulier un procédé de fabrication par thermoformage, d'une composition sous forme d'un extrudat thermoformé selon l'invention, caractérisé en ce qu'il comprend les étapes suivantes :
a) une étape d'amenée simultanée ou différée dans le temps d'au moins un triterpène et/ou d'au moins un triterpénoïde et/ou d'au moins une de leurs formes glycosylées et d'au moins un polymère choisi dans le groupe constitué des protéines naturelles ou synthétiques, des oligosaccharides naturels ou synthétiques, des polysaccharides naturels ou synthétiques, de leurs dérivés et de leurs mélanges, pour alimenter un extrudeur,
b) une étape de mélange, dans ledit extrudeur, dudit au moins un triterpène et/ou dudit au moins un triterpénoïde et/ou de ladite au moins une de leurs formes glycosylées et dudit au moins un polymère choisi dans le groupe constitué des protéines naturelles ou synthétiques, des oligosaccharides naturels ou synthétiques, des polysaccharides naturels ou synthétiques, de leurs dérivés et de leurs mélanges, pour former un mélange, et
c) une étape d'extrusion à chaud dudit mélange obtenu à l'étape b) dans ledit extrudeur pour obtenir un extrudat thermoformé dans lequel ledit au moins un triterpène et/ou ledit au moins un triterpénoïde et/ou ladite au moins une de leurs formes glycosylées comprend/ comprennent au moins une première phase amorphe et éventuellement une deuxième phase cristalline.

Un tel procédé selon l'invention donne lieu à une composition sous forme d'un extrudat thermoformé, c'est-à-dire obtenu par thermoformage et plus particulièrement par extrusion à chaud, dans lequel ledit au moins un triterpène et/ou ledit au moins un triterpénoïde et/ou ladite au moins une de leurs formes glycosylées comme principe(s) actif(s) comprend (comprennent) au moins une première phase amorphe et éventuellement une deuxième phase cristalline dispersée(s) au sein dudit au moins un polymère choisi dans le groupe constitué des protéines naturelles ou synthétiques, des oligosaccharides naturels ou synthétiques, des polysaccharides naturels ou synthétiques, de leurs dérivés et de leurs mélanges. Cette composition suivant l'invention présente une (des) solubilité(s) et/ou une (des) dispersion(s) en phase aqueuse (milieu aqueux) nettement supérieure(s) dudit au moins un triterpène et/ou dudit au moins un triterpénoïde et/ou de ladite au moins une de leurs formes glycosylées et simultanément une (des) biodisponibilité(s) significativement augmentée(s) dudit au moins un triterpène et/ou dudit au moins un triterpénoïde et/ou de ladite au moins une de leurs formes glycosylées par rapport aux solubilités et aux biodisponibilités de ces composés pour les compositions actuelles. Il a été montré que la composition selon l'invention se présente sous forme d'un extrudat thermoformé dans lequel ledit au moins un triterpène et/ou ledit au moins un triterpénoïde et/ou ladite au moins une de leurs formes glycosylées (principe(s) actif(s)) comprenant au moins une première phase amorphe et éventuellement une deuxième phase cristalline est (sont) dispersé(s) au sein dudit au moins un polymère choisi dans le groupe constitué des protéines naturelles ou synthétiques, des oligosaccharides naturels ou synthétiques, des polysaccharides naturels ou synthétiques, de leurs dérivés et de leurs mélanges.

Il a également été montré, dans le cadre de la présente invention, que ledit au moins un triterpène et/ou ledit au moins un triterpénoïde et/ou ladite au moins une de leurs formes glycosylées n'est (ne sont) pas dégradé(s) même lors du procédé de fabrication, en particulier lors du procédé de fabrication par thermoformage, de la composition sous forme d'un extrudat thermoformé qui implique pourtant la soumission dudit au moins un triterpène et/ou dudit au moins un triterpénoïde et/ou de ladite au moins une de leurs formes glycosylées à de hautes températures (HME). Par ailleurs, il a également été mis en avant que ledit au moins un triterpène et/ou ledit au moins un triterpénoïde et/ou ladite au moins une de leurs formes glycosylées dans une composition sous forme d'un extrudat thermoformé suivant l'invention y est (sont) réparti(s) (distribué(s)) de façon homogène.

Plus particulièrement, l'extrusion à chaud (Hot Melt Extrusion - HME) réalisée selon le procédé de fabrication par thermoformage suivant l'invention donne lieu à une fonte du (des) principe(s) actif(s) (ledit au moins un triterpène et/ou ledit au moins un triterpénoïde et/ou ladite au moins une de leurs formes glycosylées) et/ou dudit au moins un polymère à une température supérieure ou égale à leur point de fusion.

Toutefois, selon certains modes de réalisation d'une composition selon l'invention, cette fonte du (des) principe(s) actif(s) et/ou du polymère peut avoir lieu à une température inférieure à leur point de fusion. Ceci est par exemple le cas si la composition selon l'invention comprend un agent plastifiant ou si le (les) principe(s) actif(s) lui-même (eux-mêmes) présente (présentent) des propriétés plastifiantes. Une telle fonte du (des) principe(s) actif(s) et/ou du polymère donne lieu à une dispersion solide dans laquelle le (les) principe(s) actif(s) (ledit au moins un triterpène et/ou ledit au moins un triterpénoïde et/ou ladite au moins une de leurs formes glycosylées) comprenant au moins une première phase amorphe et éventuellement une deuxième phase cristalline est (sont) dispersé(s)/réparti(s)/distribué(s) de façon homogène au sein dudit au moins un polymère choisi dans le groupe constitué des protéines naturelles ou synthétiques, des oligosaccharides naturels ou synthétiques, des polysaccharides naturels ou synthétiques, de leurs dérivés et de leurs mélanges.

Avantageusement, le procédé suivant l'invention comprend une étape préalable de pré-mélange dudit au moins un triterpène et/ou dudit au moins un triterpénoïde et/ou de ladite au moins une de leurs formes glycosylées et dudit au moins un polymère choisi dans le groupe constitué des protéines naturelles ou synthétiques, des oligosaccharides naturels ou synthétiques, des polysaccharides naturels ou synthétiques, de leurs dérivés et de leurs mélanges, de telle sorte à former un pré-mélange destiné à alimenter l'extrudeur.

De façon préférée, selon le procédé suivant l'invention, ladite étape d'extrusion à chaud est réalisée à une température d'extrusion ou température de thermoformage comprise entre 20 et 300°C, de préférence à une température comprise entre 40 et 270°C, préférentiellement à une température comprise entre 50 et 250°C, de préférence à une température comprise entre 60 et 230°C, plus préférentiellement à une température comprise entre 70 et 220°C, plus préférentiellement à une température comprise entre 80 et 200°C, plus préférentiellement à une température comprise entre 90 et 180°C, plus préférentiellement à une température comprise entre 100 et 170°C, plus préférentiellement à une température comprise entre 120 et 160°C, plus préférentiellement à une température comprise entre 125 et 150°C.

Avantageusement, selon le procédé suivant l'invention, ladite étape d'extrusion à chaud est réalisée à une vitesse de rotation d'une vis d'extrusion comprise entre 20 et 900 tours/min, de préférence comprise entre 50 et 300 tours/min, de préférence comprise entre 100 et 250 tours/min, préférentiellement égale à 250 tours/min, préférentiellement égale à 100 tours/min.

Préférentiellement, le procédé selon l'invention comprend une étape additionnelle de refroidissement en sortie de l'extrudeur.

Avantageusement, le procédé selon l'invention comprend une étape additionnelle de traitement de l'extrudat thermoformé en sortie de l'extrudeur, par exemple une découpe au niveau d'un pelletiseur et/ou un broyage dudit extrudat thermoformé.

D'autres formes de réalisation du procédé suivant l'invention sont indiquées dans les revendications annexées.

La présente invention porte également sur une composition sous forme d'un extrudat thermoformé obtenu suivant le procédé selon l'invention, ladite composition comprenant au moins un triterpène et/ou au moins un triterpénoïde et/ou au moins une de leurs formes glycosylées comme principe actif et au moins un polymère choisi dans le groupe constitué des protéines naturelles ou synthétiques, des oligosaccharides naturels ou synthétiques, des polysaccharides naturels ou synthétiques, de leurs dérivés et de leurs mélanges, ledit au moins un triterpène et/ou ledit au moins un triterpénoïde et/ou ladite au moins une de leurs formes glycosylées comprenant au moins une première phase amorphe et éventuellement une deuxième phase cristalline.

En d'autres termes, la présente invention porte également sur une composition sous forme d'un extrudat thermoformé obtenu par thermoformage, en particulier par extrusion à chaud (HME - Hot Melt Extrusion), ladite composition comprenant au moins un triterpène et/ou au moins un triterpénoïde et/ou au moins une de leurs formes glycosylées comme principe actif et au moins un polymère choisi dans le groupe constitué des protéines naturelles ou synthétiques, des oligosaccharides naturels ou synthétiques, des polysaccharides naturels ou synthétiques, de leurs dérivés et de leurs mélanges, ledit au moins un triterpène et/ou ledit au moins un triterpénoïde et/ou ladite au moins une de leurs formes glycosylées comprenant au moins une première phase amorphe et éventuellement une deuxième phase cristalline.

La présente invention porte également sur une utilisation d'une composition suivant l'invention en tant que complément alimentaire et/ou en tant que produit cosmétique et/ou en tant que médicament à usage humain ou vétérinaire.

Une composition selon l'invention présente préférentiellement des propriétés anti-inflammatoires, hypolipémiantes, antioxydantes, antithrombotiques, antitumorales, antidiabétiques ainsi que des propriétés neuroprotectrices.

En particulier, la présente invention porte sur une composition pour utilisation dans le traitement préventif et/ou curatif, chez l'être humain et/ou chez l'animal, de pathologies en lien avec les inflammations (arthrose, tendinite, blessures, ...), de pathologies en lien avec le vieillissement prématuré des cellules, de pathologies en lien avec le système cardiovasculaire (hypotension, hypertension, vasoconstriction, hypertrophie ventriculaire, arrythmie, stéatose hépatique,...), de pathologies en lien avec le système sanguin (cholestérolémie, agrégation plaquettaire, ...), de pathologies en lien avec le système gastro-intestinal (diarrhées, inflammations digestives, modulation du microbiote intestinal, ...), de pathologies en lien avec le système endocrinien (hyperglycémie, ...), de pathologies en lien avec le système immunitaire, de pathologies en lien avec le système nerveux central, de maladies de la peau, de maladies dues à la présence de microorganismes et des cancers (anti-tumoral, ...) et dans le traitement préventif et/ou curatif du diabète.

Plus particulièrement, la présente invention porte sur une composition pour utilisation dans le traitement préventif et/ou curatif, chez l'être humain et/ou chez l'animal, des maladies liées aux articulations, aux muscles et aux tendons, des maladies liées au vieillissement prématuré des cellules, de l'obésité, du diabète, de l'hypercholestérolémie, du syndrome métabolique et du côlon irritable (IBS).

D'autres formes d'utilisation d'une composition suivant l'invention sont indiquées dans les revendications annexées.

D'autres caractéristiques, détails et avantages de l'invention ressortiront des exemples donnés ci-après, à titre non limitatif et en faisant référence aux figures annexées.
La figure 1 est un graphique illustrant le taux de solubilisation d'acides boswelliques au cours du temps pour différents exemples de compositions, en particulier pour différents exemples de compositions thermoformées, selon l'invention.
Les figures 2 et 3 sont des graphiques illustrant les taux de dispersion au cours du temps d'exemples de différentes compositions, en particulier pour des exemples de différentes compositions thermoformées, selon l'invention.
La figure 4 est un graphique illustrant le taux de solubilisation de bacosides au cours du temps pour différents exemples de compositions, en particulier pour différents exemples de compositions thermoformées, selon l'invention.
La figure 5 est un graphique illustrant le taux de solubilisation de l'asiaticoside au cours du temps pour différents exemples de compositions, en particulier pour différents exemples de compositions thermoformées, selon l'invention.
La figure 6 est un graphique illustrant le taux de dispersion de l'acide ursolique au cours du temps pour différents exemples de compositions, en particulier pour différents exemples de compositions thermoformées, selon l'invention.

### Exemples

### Exemple 1 : procédé de fabrication par thermoformage d'une composition selon l'invention sous forme d'un extrudat thermoformé

Des compositions thermoformées selon l'invention comprenant au moins un triterpène et/ou au moins un triterpénoïde et/ou au moins une de leurs formes glycosylées comprenant au moins une phase amorphe, telles que celles faisant l'objet de l'exemple 2 ci-dessous, ont été obtenues selon le procédé suivant faisant également l'objet de la présente invention :
a) une étape de pré-mélange d'au moins un triterpène et/ou d'au moins un triterpénoïde et/ou d'au moins une de leurs formes glycosylées à l'état cristallin sous forme de poudre et d'au moins un polymère choisi dans le groupe constitué des protéines naturelles ou synthétiques, des oligosaccharides naturels ou synthétiques, des polysaccharides naturels ou synthétiques, de leurs dérivés et de leurs mélanges;
b) une étape d'amenée dudit pré-mélange formé à l'étape a) pour alimenter un extrudeur de type Pharma 11 de Thermo-Fischer^{®} ;
c) une étape de mélange, dans ledit extrudeur, dudit pré-mélange pour obtenir un mélange ;
d) une étape de thermoformage par extrusion à chaud dudit mélange obtenu à l'étape c) dans ledit extrudeur pour obtenir un extrudat, thermoformé, l'étape d'extrusion à chaud étant réalisée à une vitesse de rotation d'une vis d'extrusion de 100 tours/minute et à une température comprise entre 40°C et 180°C ;
e) une étape de refroidissement en sortie de l'extrudeur dudit extrudat thermoformé obtenu à l'étape d) ; et
f) une étape de découpe/broyage, au niveau d'un broyeur, de l'extrudat thermoformé refroidi obtenu à l'étape e) de telle sorte à obtenir une poudre homogène.

La température d'extrusion à chaud (température de thermoformage) à laquelle est réalisée l'étape d'extrusion à chaud est déterminée par le type de constituants mis en œuvre, en particulier selon le type de polymère et/ou d'agent plastifiant mis en œuvre, ce que l'homme de métier est à même de déterminer. Par ailleurs, un homme de métier, notamment selon le type d'extrudeur employé et conformément au principe général de l'extrusion à chaud (HME), est à même de définir d'éventuels paliers de températures en différentes zones le long de la ou des vis d'extrusion de telle sorte qu'ait lieu une augmentation progressive de la température au sein de la matière transportée par la ou des vis d'extrusion, ceci dans un sens d'avancement de la matière au sein de l'extrudeur. Typiquement, entre zones définies le long de la ou des vis d'extrusion, des différences de températures de l'ordre de 0 à 40°C sont observées. Par exemple, dans le cadre de la présente invention, les compositions testées ci-après ont été obtenues dans un extrudeur de type Pharma 11 de Thermo-Fischer^{®} présentant 9 zones de températures qui sont les suivantes dans un sens d'avancée de la matière évoluant à une vitesse de 100 tours/minute : zone 1 (zone d'alimentation de l'extrudeur) = température ambiante ; zone 2 = 120°C ; zone 3 = 120°C; zone 4 = 120°C ; zone 5 = 130°C ; zone 6 = 140°C ; zone 7 = 150°C ; zone 8 = 155°C ; zone 9 (filière) = 160°C.

### Exemple 2 : test de solubilité de compositions thermoformées selon l'invention comprenant un extrait de Boswellia serrata standardisé à 65% d'acides boswelliques

Différentes compositions thermoformées, obtenues selon le procédé de fabrication décrit à l'exemple 1, ont été testées en termes de solubilité des acides boswelliques présents dans un extrait de Boswellia serrata standardisé à 65% d'acides boswelliques. Cette solubilité a été mesurée au cours du temps au départ des extrudats thermoformés obtenus selon l'invention. Comme indiqué ci-dessus, les extrudats thermoformés se présentent sous la forme d'une poudre homogène (broyat) dans laquelle le triterpène et/ou le triterpénoïde comprend au moins une phase amorphe.

Les tests de solubilité ont tous été réalisés avec un appareil à dissolution à palettes au départ d'environ 2 g d'extrudat thermoformé, à une température de 37°C sous agitation à 50 tours/minute dans 450 ml d'un milieu de dissolution HCl 0,1N. Ces tests de solubilité ont été menés selon les recommandations de la pharmacopée Ph.Eur.9.0 (Recommendations on Dissolution Testing). A des temps déterminés (après 30 min et après 2h), un échantillon de 1 ml de mélange a été prélevé pour réaliser un test de solubilité.

Afin de réaliser les tests de solubilité, l'échantillon testé a été filtré au travers d'un filtre (PET, taille des pores de 0,45 µm, Macherey Nagel) avant analyse HPLC (colonne Luna 5 µm C18(2) 100 A. 100*3 mm (Phenomenex) ; phase mobile : 85% A: méthanol et 15% B: eau/acétonitrile (95:5) à pH 2,8 ; débit : 0,6 mL/min ; loop : 10 µl, t° = 40°C ; longueurs d'onde : 210 nm et 247 nm).

En pratique, la solubilité des triterpènes et/ou des triterpénoïdes, en particulier la solubilité des acides boswelliques contenus dans un extrait de Boswellia serrata standardisé à 65% d'acides boswelliques, a été évaluée par dosage HPLC des 6 principaux acides boswelliques (l'acide α-boswellique, l'acide β-boswellique, l'acide 11-keto- β-boswellique (KBA), l'acide acétyl- 11-keto- β-boswellique (AKBA), l'acide acétyl α-boswellique, l'acide acétyl β-boswellique) présents dans cet extrait de Boswellia serrata.

Les compositions thermoformées selon l'invention reprises au Tableau 1 ont été formulées suivant le procédé de l'invention et testées en termes de solubilité au cours du temps selon le principe indiqué ci-dessus (dosage des 6 principaux acides boswelliques : les résultats présentés sont les moyennes de solubilité calculées en sommant les solubilités de chacun des 6 acides boswelliques et en divisant cette somme par 6). Un extrait seul de Boswellia serrata standardisé à 65% d'acides boswelliques sous forme native cristalline et sous forme de poudre (BW native) a été utilisée en tant que contrôle. Les quantités mentionnées au Tableau 1 sont des pourcentages en poids des composés mis en œuvre (soumis au procédé selon l'invention) par rapport au poids total de la composition.

**Tableau 1**

| | Extrait de Boswellia serrata (65%) (1) | Glycérol (2) | Protéine |
|---|---|---|---|
| Compo 1 | 20 | 10 | 70 (3) |
| Compo 2 | 20 | 10 | 70 (4) |
| Compo 3 | 20 | 10 | 70 (5) |

| | | | |
|---|---|---|---|
| (1) Extrait sec de Boswellia serrata standardisé à 65% d'acides boswelliques (Vidya Herbs) (2) Glycérol (Sigma-Aldrich) (3) Collagène de poisson présentant un poids moléculaire de 3000 Da (Green Snow) (4) Collagène de poisson hydrolysé présentant un poids moléculaire inférieur à 3000 Da (Kenney & Ross Ltd.) (5) Gélatine bovine présentant un poids moléculaire compris entre 1000 et 3000 Da (Lapi geltine S.P.A) | | | |

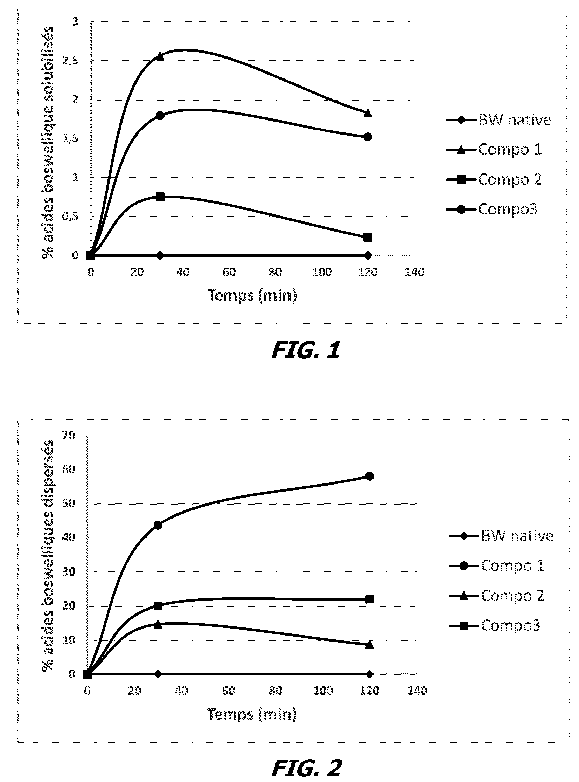

Les résultats obtenus sont présentés à la figure 1. Comme on peut le constater, les protéines naturelles de type collagène/gélatine, utilisées en tant que polymère permettent d'augmenter la solubilité des triterpènes et des triterpénoïdes, en particulier des acides boswelliques. Notons que la solubilité des acides boswelliques varie en fonction du type/nature de collagène/gélatine mais que cette solubilité est toujours augmentée par rapport au contrôle.

### Exemple 3 : test de dispersion de compositions thermoformées selon l'invention comprenant un extrait de Boswellia serrata standardisé à 65% d'acides boswelliques

Différentes compositions thermoformées, obtenues selon le procédé de fabrication décrit à l'exemple 1, ont été testées en termes de dispersion des acides boswelliques présents dans un extrait de Boswellia serrata standardisé à 65% d'acides boswelliques. La dispersion a été mesurée au cours du temps au départ des extrudats thermoformés obtenus selon l'invention. Comme indiqué ci-dessus, les extrudats thermoformés se présentent sous la forme d'une poudre homogène (broyat) dans laquelle le triterpène et/ou le triterpénoïde comprend au moins une phase amorphe.

Les tests de dispersion ont tous été réalisés avec un appareil à dissolution à palettes au départ d'environ 2 g d'extrudat thermoformé, à une température de 37°C sous agitation à 50 tours/minute dans 450 ml d'un milieu de dissolution HCl 0,1N.

Afin de réaliser les tests de dispersion, des échantillons prélevés à des temps déterminés (après 30 min et après 2h) ont été dilués dans un solvant approprié (phase mobile pour l'HPLC) puis filtré au travers d'un filtre (PET, taille des pores de 0,45 µm, Macherey Nagel) avant analyse HPLC (colonne Luna 5 µm C18(2) 100 A. 100*3 mm (Phenomenex) ; phase mobile : 85% A: méthanol et 15% B: eau/acétonitrile (95:5) à pH 2,8 ; débit : 0,6 mL/min ; loop : 10 µl, t° = 40°C ; longueurs d'onde : 210 nm et 247 nm).

En pratique, la dispersion des triterpènes et/ou des triterpénoïdes, en particulier la dispersion des acides boswelliques contenus dans un extrait de Boswellia serrata standardisé à 65% d'acides boswelliques, a été évaluée par dosage HPLC des 6 principaux acides boswelliques (l'acide α-boswellique, l'acide β-boswellique, l'acide 11-keto- β-boswellique (KBA), l'acide acétyl- 11-keto- β-boswellique (AKBA), l'acide acétyl α-boswellique, l'acide acétyl β-boswellique) présents dans cet extrait de Boswellia serrata.

Les compositions thermoformées selon l'invention reprises au Tableau 2 ont été formulées suivant le procédé de l'invention et testées en termes de dispersion aqueuse au cours du temps selon le principe indiqué ci-dessus (dosage des 6 principaux acides boswelliques : les résultats présentés sont les moyennes de dispersion calculées en sommant les dispersions de chacun des 6 acides boswelliques et en divisant cette somme par 6). Un extrait seul de Boswellia serrata standardisé à 65% d'acides boswelliques sous forme native cristalline et sous forme de poudre (BW native) a été utilisée en tant que contrôle. Les quantités mentionnées au Tableau 2 sont des pourcentages en poids des composés mis en œuvre (soumis au procédé selon l'invention) par rapport au poids total de la composition.

**Tableau 2**

| | Extrait de Boswellia serrata (65%) (1) | Glycérol (2) | Protéine | Polysaccharide |
|---|---|---|---|---|
| Compo 1 | 25 | 20 | 0 | 55 (3) |
| Compo 2 | 25 | 20 | 70 (4) | 0 |
| Compo 3 | 25 | 20 | 10 (5) | 45 (3) |
| Compo 4 | 35 | 10 | 55 (6) | 0 |
| Compo 5 | 35 | 10 | 47 (6) | 8 (3) |

| | | | | |
|---|---|---|---|---|
| (1) Extrait sec de Boswellia serrata standardisé à 65% d'acides boswelliques (Vidya Herbs) (2) Glycérol (Sigma-Aldrich) (3) Amidon modifié Cleargum CB90 (Roquette) (4) Protéine de riz (Green Snow) (5) Protéine de graines de potiron (Green Snow) (6) Collagène hydrolisé-5000 Da (Rousselot) | | | | |

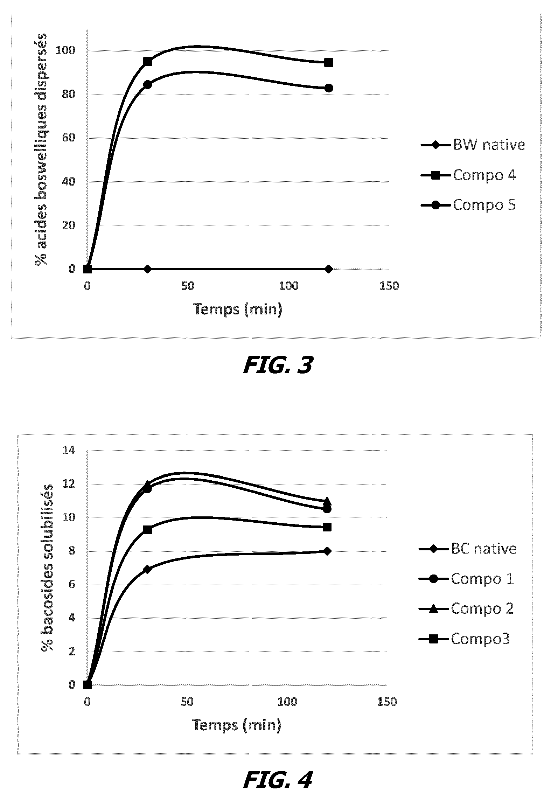

Les résultats obtenus sont présentés à la figure 2 pour les compositions 1 à 3 et à la figure 3 pour les compositions 4 et 5. Comme on peut le constater, chacune des compositions thermoformées selon l'invention donne lieu à un pourcentage de dispersion significativement supérieur à celui observé pour l'extrait seul de Boswellia serrata standardisé à 65% d'acides boswelliques sous forme native cristalline et sous forme de poudre (BW native).

### Exemple 4 : test de solubilité de compositions thermoformées selon l'invention comprenant un extrait de Bacopa monierii standardisé à 20% de bacosides

Différentes compositions thermoformées, obtenues selon le procédé de fabrication décrit à l'exemple 1, ont été testées en termes de solubilité des bacosides présents dans un extrait de Bacopa monierii standardisé à 20% de bacosides. Cette solubilité a été mesurée au cours du temps au départ des extrudats thermoformés obtenus selon l'invention. Comme indiqué ci-dessus, les extrudats thermoformés se présentent sous la forme d'une poudre homogène (broyat) dans laquelle le triterpène et/ou le triterpénoïde comprend au moins une phase amorphe.

Les tests de solubilité ont tous été réalisés avec un appareil à dissolution à palettes au départ d'environ 4 g d'extrudat thermoformé, à une température de 37°C sous agitation à 50 tours/minute dans 900 ml d'un milieu de dissolution HCl 0,1N. Ces tests de solubilité ont été menés selon les recommandations de la pharmacopée Ph.Eur.9.0 (Recommendations on Dissolution Testing). A des temps déterminés (après 30 min et après 2h), un échantillon de 1 ml de mélange a été prélevé pour réaliser un test de solubilité.

Afin de réaliser les tests de solubilité, l'échantillon testé a été centrifugé (10000 tours/minute durant 10 min à température ambiante, Microstar 17(VWR)), le surnageant a été filtré au travers d'un filtre (PET, taille des pores de 0,45 µm, Macherey Nagel) avant analyse HPLC (colonne C18, 5 µm 250*4.6 mm (Agilent) ; phase mobile : A : Tampon phosphate 0,1 mM et B : Acétonitrile selon le gradient suivant :

| **TEMPS (MIN)** | **A** | **B** |
|---|---|---|
| 0 | 70% | 30% |
| 25 | 60% | 40% |
| 26 | 70% | 30% |
| 30 | 70% | 30% |

| | | |
|---|---|---|
| Débit : 1,5 ml/min ; loop, 20µl ; t° : 25°C ; longueur d'onde 205 nm). | | |

En pratique, la solubilité des triterpènes et/ou des triterpénoïdes, en particulier la solubilité des bacosides contenus dans un extrait de Bacopa monierii standardisé à 20% de bacosides, a été évaluée par dosage HPLC des 5principaux bacosides (Bacopaside I, Bacoside A3, Bacopaside II, Jujubogenin, Bacopasaponin C) présents dans cet extrait de Bacopa monierii.

Les compositions thermoformées selon l'invention reprises au Tableau 3 ont été formulées suivant le procédé de l'invention et testées en termes de solubilité au cours du temps selon le principe indiqué ci-dessus (dosage des 5 principaux bacosides : les résultats présentés sont les moyennes de solubilité calculées en sommant les solubilités de chacun des 5 bacosides et en divisant cette somme par 5). Un extrait seul de Bacopa monierii standardisé à 20% de bacosides sous forme native cristalline et sous forme de poudre (BC native) a été utilisée en tant que contrôle. Les quantités mentionnées au Tableau 3 sont des pourcentages en poids des composés mis en œuvre (soumis au procédé selon l'invention) par rapport au poids total de la composition.

**Tableau 3**

| | Extrait de Bacopa monierii (20%) (1) | Glycérol (2) | Protéine (3) | Polysaccharide (4) |
|---|---|---|---|---|
| Compo 1 | 33 | 14 | 53 | 0 |
| Compo 2 | 33 | 14 | 43 | 10 |
| Compo 3 | 25 | 14 | 0 | 53 |

| | | | | |
|---|---|---|---|---|
| (1) Extrait sec de Bacopa Monierii standardisé à 20% de bacosides (Vidya Herbs) (2) Glycérol (Sigma-Aldrich) (3) Collagène hydrolysé présentant un poids moléculaire de 5000 Da (Rousselot) (4) Tackidex C760 (Roquette) | | | | |

Les résultats obtenus sont présentés à la figure 4. Comme on peut le constater, les compositions selon l'invention permettent toutes d'augmenter le % de bacosides solubilisés par rapport au contrôle.

### Exemple 5 : test de solubilité de compositions thermoformées selon l'invention comprenant de l'asiaticoside

Différentes compositions thermoformées, obtenues selon le procédé de fabrication décrit à l'exemple 1, ont été testées en termes de solubilité de l'asiaticoside. Cette solubilité a été mesurée au cours du temps au départ des extrudats thermoformés obtenus selon l'invention. Comme indiqué ci-dessus, les extrudats thermoformés se présentent sous la forme d'une poudre homogène (broyat) dans laquelle le triterpène et/ou le triterpénoïde comprend au moins une phase amorphe.

Les tests de solubilité ont tous été réalisés avec un appareil à dissolution à palettes au départ d'environ 2 g d'extrudat thermoformé, à une température de 37°C sous agitation à 50 tours/minute dans 900 ml d'un milieu de dissolution HCl 0,1N. Ces tests de solubilité ont été menés selon les recommandations de la pharmacopée Ph.Eur.9.0 (Recommendations on Dissolution Testing). A des temps déterminés (après 30 min et après 2h), un échantillon de 1 ml de mélange a été prélevé pour réaliser un test de solubilité.

Afin de réaliser les tests de solubilité, l'échantillon testé a été centrifugé (10000 tours/minute durant 10 min à température ambiante, Microstar 17(VWR)), le surnageant a été filtré au travers d'un filtre (PET, taille des pores de 0,45 µm, Macherey Nagel) avant analyse HPLC (colonne C18, 5µm 250*4.6 mm (Agilent) ; phase mobile : A : Eau et B : Acétonitrile selon le gradient suivant :

| **TEMPS (MIN)** | **A** | **B** |
|---|---|---|
| 0 | 80% | 20% |
| 18 | 59% | 41% |
| 20 | 45% | 55% |
| 23 | 45% | 55% |
| 28 | 80% | 20% |
| 33 | 80% | 20% |

| | | |
|---|---|---|
| Débit : 1 ml/min ; loop, 10µl ; t° : 25°C ; longueur d'onde 205 nm). | | |

En pratique, la solubilité des triterpènes et/ou des triterpénoïdes, en particulier la solubilité de l'asiaticoside, a été évaluée par dosage HPLC.

Les compositions thermoformées selon l'invention reprises au Tableau 4 ont été formulées suivant le procédé de l'invention et testées en termes de solubilité au cours du temps selon le principe indiqué ci-dessus. De l'asiaticoside sous forme native cristalline et sous forme de poudre (AS native) a été utilisée en tant que contrôle. Les quantités mentionnées au Tableau 4 sont des pourcentages en poids des composés mis en œuvre (soumis au procédé selon l'invention) par rapport au poids total de la composition.

**Tableau 4**

| | Asiaticoside (1) | Glycérol (2) | Protéine (3) | Polysaccharide (4) |
|---|---|---|---|---|
| Compo 1 | 10 | 10 | 70 | 10 |
| Compo 2 | 10 | 14 | 0 | 76 |

| | | | | |
|---|---|---|---|---|
| (1) Asiaticoside (FyzCo) (2) Glycérol (Sigma-Aldrich) (3) Collagène hydrolysé présentant un poids moléculaire de 5000 Da (Rousselot) (4) Tackidex C760 (Roquette) | | | | |

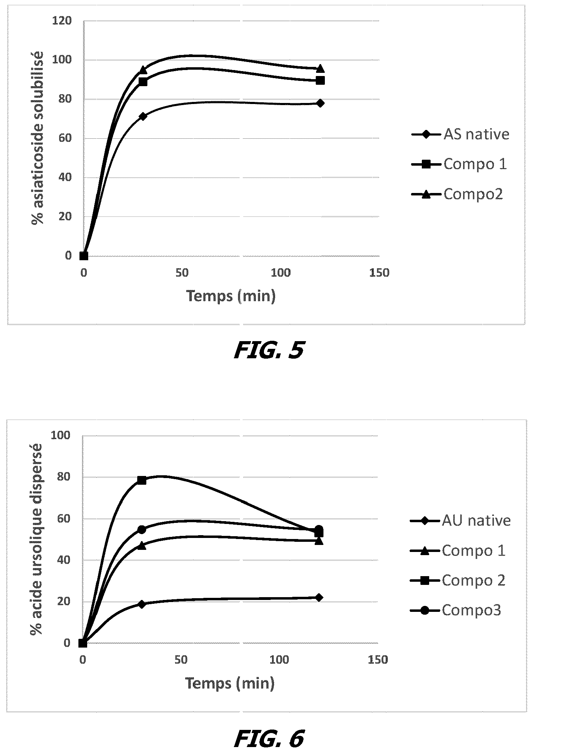

Les résultats obtenus sont présentés à la figure 5. Comme on peut le constater, les compositions selon l'invention permettent toutes d'augmenter le % d'asiaticoside solubilisé par rapport au contrôle.

### Exemple 6 : test de dispersion de compositions thermoformées selon l'invention comprenant de l'acide ursolique

Différentes compositions thermoformées, obtenues selon le procédé de fabrication décrit à l'exemple 1, ont été testées en termes de dispersion d'acide ursolique. La dispersion a été mesurée au cours du temps au départ des extrudats thermoformés obtenus selon l'invention. Comme indiqué ci-dessus, les extrudats thermoformés se présentent sous la forme d'une poudre homogène (broyat) dans laquelle le triterpène et/ou le triterpénoïde comprend au moins une phase amorphe.

Les tests de dispersion ont tous été réalisés avec un appareil à dissolution à palettes au départ d'environ 2g d'extrudat thermoformé, à une température de 37°C sous agitation à 50 tours/minute dans 900 ml d'un milieu de dissolution HCl 0,1N.

Afin de réaliser les tests de dispersion, des échantillons prélevés à des temps déterminés (après 30 min et après 2h) ont été dilués dans un solvant approprié (phase mobile pour l'HPLC) puis filtré au travers d'un filtre (PET, taille des pores de 0,45 µm, Macherey Nagel) avant analyse HPLC (colonne Luna 5 µm C18(2) 100 A. 100*3 mm (Phenomenex) ; phase mobile Acétonitrile/Eau/0,5% Acétate d'ammonium (67 :12 :21) débit : 1 mL/min ; loop : 10 µl, t° = 25°C ; longueurs d'onde : 210 nm).

En pratique, la dispersion des triterpènes et/ou des triterpénoïdes, en particulier la dispersion de l'acide ursolique, a été évaluée par dosage HPLC.

Les compositions thermoformées selon l'invention reprises au Tableau 5 ont été formulées suivant le procédé de l'invention et testées en termes de dispersion aqueuse au cours du temps selon le principe indiqué ci-dessus. De l'acide ursolique sous forme native cristalline et sous forme de poudre (AU native) a été utilisée en tant que contrôle. Les quantités mentionnées au Tableau 5 sont des pourcentages en poids des composés mis en œuvre (soumis au procédé selon l'invention) par rapport au poids total de la composition.

**Tableau 5**

| | Acide ursolique (1) | Glycérol (2) | Protéine (3) | Polysaccharide (4) |
|---|---|---|---|---|
| Compo 1 | 10 | 10 | 80 | 0 |
| Compo 2 | 10 | 14 | 66 | 10 |
| Compo 3 | 10 | 14 | 0 | 76 |

| | | | | |
|---|---|---|---|---|
| (1) Acide ursolique (Fyzco) (2) Glycérol (Sigma-Aldrich) (3) Collagène hydrolysé présentant un poids moléculaire de 5000 Da (Rousselot) (4) Tackidex C760 (Roquette) | | | | |

Les résultats obtenus sont présentés à la figure 6. Comme on peut le constater, les compositions selon l'invention permettent toutes d'augmenter le % d'acide ursolique dispersé par rapport au contrôle.

La présente invention a été décrite en relation avec des modes de réalisations spécifiques, qui ont une valeur purement illustrative et ne doivent pas être considérés comme limitatifs. D'une manière générale, il apparaîtra évident pour l'homme du métier que la présente invention n'est pas limitée aux exemples illustrés et/ou décrits ci-dessus.

L'usage des verbes « comprendre », « inclure », « comporter », ou toute autre variante, ainsi que leurs conjugaisons, ne peut en aucune façon exclure la présence d'éléments autres que ceux mentionnés.

L'usage de l'article indéfini « un », « une », ou de l'article défini « le », « la » ou « l' », pour introduire un élément n'exclut pas la présence d'une pluralité de ces éléments.

## Revendications

1. Composition sous forme d'un extrudat thermoformé, éventuellement conditionné en pellet, en flake, en granulé, en poudre, en comprimé effervescent ou non, en solution injectable ou non, en suspension, en gel, en pommade ou encore sous toute autre forme adéquate permettant une administration à un animal ou à un être humain, comprenant au moins un triterpène et/ou au moins un triterpénoïde et/ou au moins une de leurs formes glycosylées et au moins un polymère choisi dans le groupe constitué des protéines naturelles ou synthétiques, des oligosaccharides naturels ou synthétiques, des polysaccharides naturels ou synthétiques, et de leurs mélanges, ledit au moins un triterpène et/ou ledit au moins un triterpénoïde et/ou ladite au moins une de leurs formes glycosylées comprenant au moins une première phase amorphe et éventuellement une deuxième phase cristalline.

2. Composition selon la revendication 1, **caractérisée en ce que** ledit extrudat thermoformé comprend un mélange thermoformé dudit au moins un triterpène et/ou dudit au moins un triterpénoïde et/ou de ladite au moins une de leurs formes glycosylées et dudit au moins un polymère choisi dans le groupe constitué des protéines naturelles ou synthétiques, des oligosaccharides naturels ou synthétiques, des polysaccharides naturels ou synthétiques, et de leurs mélanges.

3. Composition selon la revendication 1 ou 2, **caractérisée en ce que** ledit au moins un triterpène et/ou ledit au moins un triterpénoïde et/ou ladite au moins une de leurs formes glycosylées comprend majoritairement au moins une première phase amorphe.

4. Composition selon la revendication 3, **caractérisée en ce que** ledit au moins un triterpène et/ou ledit au moins un triterpénoïde et/ou ladite au moins une de leurs formes glycosylées comprend entre 51 et 100% en masse d'une phase amorphe et entre 0 et 49% en masse d'une phase cristalline.

5. Composition selon l'une quelconque des revendications précédentes, **caractérisée en ce que** ledit au moins un triterpène et/ou ledit au moins un triterpénoïde et/ou ladite au moins une de leurs formes glycosylées est tétracyclique, comme par exemple l'oléandrine, l'euphol ou la cucurbitacine, ou pentacyclique, comme par exemple l'acide bétulinique, l'acide oléanolique, un acide boswellique, l'acide ursolique, le lupéol, l'acide asiatique, l'acide madécassique, l'acide maslinique, la jujubogenine ou la pseudojujubogenine.

6. Composition selon la revendication 5, **caractérisée en ce que** ledit acide boswellique est choisi dans le groupe constitué des acides α- et β-boswelliques, par exemple l'acide α-boswellique, l'acide acétyl-α-boswellique, l'acide β-boswellique, l'acide acétyl-β-boswellique, l'acide 9,11-dehydro-α-boswellique, l'acide acétyl-9,11-dehydro-α-boswellique, l'acide 9,11-dehydro-β-boswellique, l'acide acétyl-9,11-dehydro-β-boswellique, 11-céto-β-boswellique, l'acide 11-céto- α-boswellique, l'acide 3-acétyl-11-céto-α-boswellique et l'acide 3-acétyl-11-céto-β-boswellique.

7. Composition selon l'une quelconque des revendications précédentes, **caractérisée en ce que** lesdites protéines naturelles ou synthétiques sont choisies dans le groupe constitué des glycoprotéines, des collagènes et/ou des hydrolysats de collagène, des protéines végétales, des protéines animales, et de leurs mélanges.

8. Composition selon la revendication 7, **caractérisée en ce que** lesdits collagènes et/ou lesdits hydrolysats de collagène présentent un poids moléculaire compris entre 50 et 300000 Da, de préférence entre 100 et 275000 Da, de préférence entre 150 et 250000 Da, de préférence entre 200 et 225000 Da, de préférence entre 250 et 200000 Da, de préférence entre 300 et 175000 Da, de préférence entre 350 et 150000 Da, de préférence entre 400 et 125000 Da, de préférence entre 450 et 100000 Da, de préférence entre 500 et 75000 Da, de préférence entre 550 et 50000 Da, de préférence entre 600 et 40000 Da, de préférence entre 650 et 30000 Da, de préférence entre 700 et 20000 Da, de préférence entre 750 et 10000 Da, de préférence entre 800 et 9000 Da, de préférence entre 850 et 8000 Da, de préférence entre 900 et 7000 Da, de préférence entre 950 et 6000 Da, de préférence entre 1000 et 5000 Da, de préférence entre 1050 et 4000 Da, de préférence entre 1100 et 3000 Da, de préférence entre 1150 et 2000 Da, de préférence entre 1200 et 1000 Da.

9. Composition selon l'une quelconque des revendications précédentes, **caractérisée en ce que** lesdits oligosaccharides sont choisis dans le groupe constitué de la cyclodextrine, du raffinose, du rhamminose, du rhamnose, du stachyose, du verbascose, du tréhalose, du lactose, du lactulose, du maltose, et de leurs mélanges.

10. Composition selon l'une quelconque des revendications précédentes, **caractérisée en ce que** lesdits polysaccharides naturels ou synthétiques sont choisis dans le groupe constitué des amidons, des fibres, des celluloses, des hémicelluloses, du glycogène, du β-glucane, de l'inuline, de l'amylopectine, de l'amylose, de la dextrine, de la maltodextrine, de l'isomaltose, du xylane, du pullulane, de l'agar-agar, des carraghénanes, des mannanes, du fucoïdane, des gommes, du chitosan, de la chitine, du xanthane, de la lévane, de la néoserine, de l'acide hyaluronique, des hyaluronates, du sulfate de chondroïtine, du dermatane sulfate, du kératane sulfate, et de leurs mélanges.

11. Composition selon l'une quelconque des revendications précédentes, **caractérisée en ce qu'**elle comprend en outre au moins un polymère naturel ou synthétique additionnel choisi dans le groupe constitué de l'acétate polyvinyle, du polyvinylpyrrolidone (PVP), de l'acétate de polyvinylpyrrolidone-co-vinyle, de l'acétate de polyethylène-co-vinyle, de l'acétate co-méthacrylique d'acide polyvinyle, du polyethylène oxide, du polylactide-co-glycolide, de l'alcool polyvinylique, du polycarbophile, de la polycaprolactone, de la cire de carnauba, du copolymère d'éthylène-vinyle, de la lécithine, de l'huile de ricin, de l'huile de soja hydrogénée, des cires, de l'isomalt, et de leurs mélanges.

12. Composition selon l'une quelconque des revendications précédentes, **caractérisée en ce qu'**elle comprend en outre au moins un agent plastifiant.

13. Composition selon la revendication 12, **caractérisée en ce que** ledit au moins un agent plastifiant est choisi dans le groupe constitué des polyols, des lipides, des esters de sucrose, de l'eau, du citrate de triéthyle, du polyéthylène glycol, du sébate de dibutyle, du stéarate de butyle, du monostéarate de glycérol, du phtalate diéthyle, et de leurs mélanges.

14. Composition selon l'une quelconque des revendications précédentes, **caractérisée en ce qu'**elle comprend en outre au moins un additif choisi dans le groupe constitué des agents lubrifiants, des agents surfactants, des agents antioxydants, des agents chélatants, et de leurs mélanges.

15. Composition selon l'une quelconque des revendications précédentes, **caractérisée en ce qu'**elle comprend en outre au moins un premier composé additionnel de type polyphénol choisi dans le groupe constitué des acides phénoliques, des stilbènes, des alcools phénoliques, des lignanes, des flavonoïdes, et de leurs mélanges.

16. Procédé de fabrication, en particulier procédé de fabrication par thermoformage, d'une composition sous forme d'un extrudat thermoformé selon l'une quelconque des revendications 1 à 15, **caractérisé en ce qu'**il comprend les étapes suivantes :
a) une étape d'amenée simultanée ou différée dans le temps d'au moins un triterpène et/ou d'au moins un triterpénoïde et/ou d'au moins une de leurs formes glycosylées et d'au moins un polymère choisi dans le groupe constitué des protéines naturelles ou synthétiques, des oligosaccharides naturels ou synthétiques, des polysaccharides naturels ou synthétiques, et de leurs mélanges, pour alimenter un extrudeur,
b) une étape de mélange, dans ledit extrudeur, dudit au moins un triterpène et/ou dudit au moins un triterpénoïde et/ou de ladite au moins une de leurs formes glycosylées et dudit au moins un polymère choisi dans le groupe constitué des protéines naturelles ou synthétiques, des oligosaccharides naturels ou synthétiques, des polysaccharides naturels ou synthétiques, et de leurs mélanges, pour former un mélange, et
c) une étape d'extrusion à chaud dudit mélange obtenu à l'étape b) dans ledit extrudeur pour obtenir un extrudat thermoformé dans lequel ledit au moins un triterpène et/ou ledit au moins un triterpénoïde et/ou ladite au moins une de leurs formes glycosylées comprend/comprennent au moins une première phase amorphe et éventuellement une deuxième phase cristalline.

17. Procédé selon la revendication 16, **caractérisé en ce qu'**il comprend une étape préalable de pré-mélange dudit au moins un triterpène et/ou dudit au moins un triterpénoïde et/ou de ladite au moins une de leurs formes glycosylées et dudit au moins un polymère choisi dans le groupe constitué des protéines naturelles ou synthétiques, des oligosaccharides naturels ou synthétiques, des polysaccharides naturels ou synthétiques, et de leurs mélanges, de telle sorte à former un pré-mélange destiné à alimenter l'extrudeur.

18. Procédé selon la revendication 16 ou 17, **caractérisé en ce que** ladite étape d'extrusion à chaud est réalisée à une température d'extrusion comprise entre 20 et 300°C, de préférence à une température comprise entre 40 et 270°C, préférentiellement à une température comprise entre 50 et 250°C, de préférence à une température comprise entre 60 et 230°C, plus préférentiellement à une température comprise entre 70 et 220°C, plus préférentiellement à une température comprise entre 80 et 200°C, plus préférentiellement à une température comprise entre 90 et 180°C, plus préférentiellement à une température comprise entre 100 et 170°C, plus préférentiellement à une température comprise entre 120 et 160°C, plus préférentiellement à une température comprise entre 125 et 150°C.

19. Procédé selon l'une quelconque des revendications 16 à 18, **caractérisé en ce que** ladite étape d'extrusion à chaud est réalisée à une vitesse de rotation d'une vis d'extrusion comprise entre 20 et 900 tours/min, de préférence comprise entre 50 et 300 tours/min, de préférence comprise entre 100 et 250 tours/min, préférentiellement égale à 250 tours/min, préférentiellement égale à 100 tours/min.

20. Procédé selon l'une quelconque des revendications 16 à 19, **caractérisé en ce qu'**il comprend une étape additionnelle de refroidissement en sortie de l'extrudeur.

21. Procédé selon l'une quelconque des revendications 16 à 20, **caractérisé en ce qu'**il comprend une étape additionnelle de traitement de l'extrudat thermoformé en sortie de l'extrudeur, par exemple une découpe au niveau d'un pelletiseur et/ou un broyage dudit extrudatthermoformé.

22. Composition sous forme d'un extrudat thermoformé selon l'une quelconque des revendications 1 à 15 pour utilisation dans le traitement préventif et/ou curatif, chez l'être humain et/ou chez l'animal, de pathologies en lien avec les inflammations, de pathologies en lien avec le vieillissement prématuré des cellules, de pathologies en lien avec le système cardiovasculaire, de pathologies en lien avec le système sanguin, de pathologies en lien avec le système gastro-intestinal, de pathologies en lien avec le système endocrinien, de pathologies en lien avec le système immunitaire, de pathologies en lien avec le système nerveux central, de maladies de la peau, de maladies dues à la présence de microorganismes et des cancers et dans le traitement préventif et/ou curatif du diabète.

23. Composition sous forme d'un extrudat thermoformé obtenu suivant le procédé selon l'une quelconque des revendications 16 à 21, ladite composition comprenant au moins un triterpène et/ou au moins un triterpénoïde et/ou au moins une de leurs formes glycosylées comme principe actif et au moins un polymère choisi dans le groupe constitué des protéines naturelles ou synthétiques, des oligosaccharides naturels ou synthétiques, des polysaccharides naturels ou synthétiques, et de leurs mélanges, ledit au moins un triterpène et/ou ledit au moins un triterpénoïde et/ou ladite au moins une de leurs formes glycosylées comprenant au moins une première phase amorphe et éventuellement une deuxième phase cristalline.

## Patentansprüche

1. Zusammensetzung in Form eines thermogeformten Extrudats, gegebenenfalls konditioniert als Pellets, Flocken, Granulat, Pulver, sprudelnde oder nicht sprudelnde Tabletten, injizierbare oder nicht injizierbare Lösung, Suspension, Gel, Salbe oder in jeder anderen geeigneten Form, die eine Verabreichung an ein Tier oder einen Menschen ermöglicht, die mindestens ein Triterpen und/oder mindestens ein Triterpenoid und/oder mindestens eine ihrer glykosylierten Formen und mindestens ein Polymer umfasst, das aus der Gruppe ausgewählt ist, die aus natürlichen oder synthetischen Proteinen, natürlichen oder synthetischen Oligosacchariden, natürlichen oder synthetischen Polysacchariden und deren Mischungen besteht, wobei das mindestens eine Triterpen und/oder das mindestens eine Triterpenoid und/oder mindestens eine ihrer glykosylierten Formen mindestens eine erste amorphe Phase und gegebenenfalls eine zweite kristalline Phase umfasst.

2. Zusammensetzung nach Anspruch 1, **dadurch gekennzeichnet, dass** das thermogeformte Extrudat eine thermogeformte Mischung aus dem mindestens einen Triterpen und/oder dem mindestens einen Triterpenoid und/oder mindestens einer ihrer glykosylierten Formen und dem mindestens einen Polymer umfasst, das aus der Gruppe ausgewählt ist, die aus natürlichen oder synthetischen Proteinen, natürlichen oder synthetischen Oligosacchariden, natürlichen oder synthetischen Polysacchariden und deren Mischungen besteht.

3. Zusammensetzung nach Anspruch 1 oder 2, **dadurch gekennzeichnet, dass** das mindestens eine Triterpen und/oder das mindestens eine Triterpenoid und/oder die mindestens eine ihrer glykosylierten Formen überwiegend mindestens eine erste amorphe Phase umfasst.

4. Zusammensetzung nach Anspruch 3, **dadurch gekennzeichnet, dass** das mindestens eine Triterpen und/oder das mindestens eine Triterpenoid und/oder die mindestens eine ihrer glykosylierten Formen zwischen 51 und 100 Massenprozent einer amorphen Phase und zwischen 0 und 40 Massenprozent einer kristallinen Phase umfasst.

5. Zusammensetzung nach einem der vorstehenden Ansprüche, **dadurch gekennzeichnet, dass** das mindestens eine Triterpen und/oder das mindestens eine Triterpenoid und/oder die mindestens eine ihrer glykosylierten Formen tetrazyklisch ist, wie beispielsweise Oleandrin, Euphol oder Cucurbitacin, oder pentazyklisch ist, wie beispielsweise Betulinsäure, Oleanolsäure, eine Boswelliasäure, Ursolsäure, Lupeol, Asiatsäure, Madekassinsäure, Maslinsäure, Jujubogenin oder Pseudojujubogenin.

6. Zusammensetzung nach Anspruch 5, **dadurch gekennzeichnet, dass** die Boswelliasäure aus der Gruppe ausgewählt ist, die aus α- und β-Boswelliasäuren, beispielsweise α-Boswelliasäure, Acetyl-α-Boswelliasäure, β-Boswelliasäure, Acetyl-β-Boswelliasäure, 9,11-Dehydro-α-Boswelliasäure, Acetyl-9,11-Dehydro-α-Boswelliasäure, 9,11-Dehydro-β-Boswelliasäure, Acetyl-9,11-Dehydro-β-Boswelliasäure, 11-Keto-β-Boswelliasäure, 11-Keto-α-Boswelliasäure, 3-Acetyl-11-Keto-α-Boswelliasäure und 3-Acetyl-11-Keto-β-Boswelliasäure besteht.

7. Zusammensetzung nach irgendeinem der vorstehenden Ansprüche, **dadurch gekennzeichnet, dass** die natürlichen oder synthetischen Proteine aus der Gruppe ausgewählt sind, die aus Glykoproteinen, Kollagenen und/oder Kollagenhydrolysaten, pflanzlichen Proteinen, tierischen Proteinen und deren Mischungen besteht.

8. Zusammensetzung nach Anspruch 7, **dadurch gekennzeichnet, dass** die Kollagene und/oder die Kollagenhydrolysate ein Molekulargewicht zwischen 50 und 300.000 Da, bevorzugt zwischen 100 und 275.000 Da, bevorzugt zwischen 150 und 250.000 Da, bevorzugt zwischen 200 und 225.000 Da, bevorzugt zwischen 250 und 200.000 Da, bevorzugt zwischen 300 und 175.000 Da, bevorzugt zwischen 350 und 150.000 Da, bevorzugt zwischen 400 und 125.000 Da, bevorzugt zwischen 450 und 100.000 Da, bevorzugt zwischen 500 und 75.000 Da, bevorzugt zwischen 550 und 50.000 Da, bevorzugt zwischen 600 und 40.000 Da, bevorzugt zwischen 650 und 30.000 Da, bevorzugt zwischen 700 und 20.000 Da, bevorzugt zwischen 750 und 10.000 Da, bevorzugt zwischen 800 und 9.000 Da, bevorzugt zwischen 850 und 8.000 Da, bevorzugt zwischen 900 und 7.000 Da, bevorzugt zwischen 950 und 6.000 Da, bevorzugt zwischen 1.000 und 5.000 Da, bevorzugt zwischen 1.050 und 4.000 Da, bevorzugt zwischen 1.100 und 3.000 Da, bevorzugt zwischen 1.150 und 2.000 Da, bevorzugt zwischen 1.200 und 1.000 Da aufweisen.

9. Zusammensetzung nach irgendeinem der vorstehenden Ansprüche, **dadurch gekennzeichnet, dass** die Oligosaccharide aus der Gruppe ausgewählt sind, die aus Cyclodextrin, Raffinose, Rhamminose, Rhamnose, Stachyose, Verbascose, Trehalose, Laktose, Laktulose, Maltose und deren Mischungen besteht.

10. Zusammensetzung nach irgendeinem der vorstehenden Ansprüche, **dadurch gekennzeichnet, dass** die natürlichen oder synthetischen Polysaccharide aus der Gruppe ausgewählt sind, die aus Stärken, Ballaststoffen, Zellulosen, Hemizellulosen, Glykogen, β-Glukan, Inulin, Amylopektin, Amylose, Dextrin, Maltodextrin, Isomaltose, Xylan, Pullulan, Agar-Agar, Carrageen, Mannane, Fucoidan, Gummi, Chitosan, Chitin, Xanthan, Levan, Neoserin, Hyaluronsäure, Hyaluronate, Chondroitinsulfat, Dermatansulfat, Keratansulfat und deren Mischungen besteht.

11. Zusammensetzung nach irgendeinem der vorstehenden Ansprüche, **dadurch gekennzeichnet, dass** sie ferner mindestens ein zusätzliches natürliches oder synthetisches Polymer umfasst, das aus der Gruppe ausgewählt ist, die aus Polyvinylazetat, Polyvinylpyrrolidon (PVP), Polyvinylpyrrolidon-co-vinylazetat, Polyethylen-co-vinylazetat, Polyvinyl-co-methacrylsäureazetat, Polyethylenoxid, Polylaktid-co-glykolid, Polyvinylalkohol, Polycarbophil, Polycaprolakton, Carnaubawachs, Ethylen-Vinyl-Copolymer, Lezithin, Rizinusöl, hydriertes Sojaöl, Wachsen, Isomalt und deren Mischungen besteht.

12. Zusammensetzung nach irgendeinem der vorstehenden Ansprüche, **dadurch gekennzeichnet, dass** sie ferner mindestens einen Weichmacher umfasst.

13. Zusammensetzung nach Anspruch 12, **dadurch gekennzeichnet, dass** der mindestens eine Weichmacher aus der Gruppe ausgewählt ist, die aus Polyolen, Lipiden, Saccharoseestern, Wasser, Triethylzitrat, Polyethylenglykol, Dibutylsebacat, Butylstearat, Glyzerinmonostearat, Diethylphthalat und deren Mischungen besteht.

14. Zusammensetzung nach irgendeinem der vorstehenden Ansprüche, **dadurch gekennzeichnet, dass** sie ferner mindestens ein Additiv umfasst, das aus der Gruppe ausgewählt ist, die aus Schmiermitteln, Tensiden, Antioxidantien, Chelatbildnern und deren Mischungen besteht.

15. Zusammensetzung nach irgendeinem der vorstehenden Ansprüche, **dadurch gekennzeichnet, dass** sie ferner mindestens eine erste zusätzliche Verbindung vom Polyphenoltyp umfasst, die aus der Gruppe ausgewählt ist, die aus Phenolsäuren, Stilbenen, Phenolalkoholen, Lignanen, Flavonoiden und deren Mischungen besteht.

16. Verfahren zur Herstellung, insbesondere Verfahren zur Herstellung durch Thermoformen, einer Zusammensetzung in Form eines thermogeformten Extrudats nach irgendeinem der Ansprüche 1 bis 15, **dadurch gekennzeichnet, dass** es die folgenden Schritte umfasst:
a) einen Schritt der gleichzeitigen oder zeitlich versetzten Zuführung von mindestens einem Triterpen und/oder mindestens einem Triterpenoid und/oder mindestens einer ihrer glykosylierten Formen und mindestens einem Polymer, ausgewählt aus der Gruppe, die aus natürlichen oder synthetischen Proteinen, natürlichen oder synthetischen Oligosacchariden, natürlichen oder synthetischen Polysacchariden sowie deren Mischungen, zur Beschickung eines Extruders besteht,
b) einen Schritt des Mischens in dem Extruder, des mindestens einen Triterpens und/oder des mindestens einen Triterpenoids und/oder der mindestens einen ihrer glykosylierten Formen und des mindestens einen Polymers, ausgewählt aus der Gruppe, die aus natürlichen oder synthetischen Proteinen, natürlichen oder synthetischen Oligosacchariden, natürlichen oder synthetischen Polysacchariden und deren Mischungen besteht, zur Bildung einer Mischung, und
c) einen Schritt des Heißextrudierens der in Schritt b) erhaltenen Mischung in dem Extruder, um ein thermogeformtes Extrudat zu erhalten, in welchem das mindestens eine Triterpen und/oder das mindestens eine Triterpenoid und/oder mindestens eine ihrer glykosylierten Formen mindestens eine erste amorphe Phase und gegebenenfalls eine zweite kristalline Phase umfasst/umfassen.

17. Verfahren nach Anspruch 16, **dadurch gekennzeichnet, dass** es einen vorangehenden Schritt des Vormischens des mindestens einen Triterpens und/oder des mindestens einen Triterpenoids und/oder der mindestens einen ihrer glykosylierten Formen und des mindestens einen Polymers ausgewählt aus der Gruppe, die aus natürlichen oder synthetischen Proteinen, natürlichen oder synthetischen Oligosacchariden, natürlichen oder synthetischen Polysacchariden und deren Mischungen besteht, umfasst, um eine Vormischung zur Beschickung des Extruders zu bilden.

18. Verfahren nach Anspruch 16 oder 17, **dadurch gekennzeichnet, dass** der Heißextrusionsschritt bei einer Extrusionstemperatur von zwischen 20 und 300 °C, bevorzugt bei einer Temperatur von zwischen 40 und 270 °C, vorzugsweise bei einer Temperatur zwischen von 50 und 250 °C, bevorzugt bei einer Temperatur von zwischen 60 und 230 °C, bevorzugter bei einer Temperatur von zwischen 70 und 220 °C, bevorzugter bei einer Temperatur von zwischen 80 und 200 °C, bevorzugter bei einer Temperatur von zwischen 90 und 180 °C, bevorzugter bei einer Temperatur von zwischen 100 und 170 °C, bevorzugter bei einer Temperatur von zwischen 120 und 160 °C, bevorzugter bei einer Temperatur von zwischen 125 und 150 °C durchgeführt wird.

19. Verfahren nach irgendeinem der Ansprüche 16 bis 18, **dadurch gekennzeichnet, dass** der Heißextrusionsschritt bei einer Drehzahl der Extrusionsschnecke von zwischen 20 und 900 U/min, bevorzugt von zwischen 50 und 300 U/min, bevorzugt von zwischen 100 und 250 U/min, vorzugsweise von gleich 250 U/min, vorzugsweise von gleich 100 U/min durchgeführt wird.

20. Verfahren nach irgendeinem der Ansprüche 16 bis 19, **dadurch gekennzeichnet, dass** es einen zusätzlichen Schritt des Abkühlens am Ausgang des Extruders umfasst.

21. Verfahren nach irgendeinem der Ansprüche 16 bis 20, **dadurch gekennzeichnet, dass** es einen zusätzlichen Schritt zur Behandlung des thermogeformten Extrudats am Ausgang des Extruders umfasst, beispielsweise ein Schneiden an einem Pelletierer und/oder ein Zerkleinern des thermogeformten Extrudats.

22. Zusammensetzung in Form eines thermogeformten Extrudats nach irgendeinem der Ansprüche 1 bis 15 zur Verwendung bei der präventiven und/oder kurativen Behandlung beim Menschen und/oder beim Tier von Erkrankungen in Verbindung mit Entzündungen, von Erkrankungen in Verbindung mit vorzeitiger Zellalterung, von Erkrankungen in Verbindung mit dem Herz-Kreislauf-System, von Erkrankungen in Verbindung mit dem Blutsystem, von Erkrankungen in Verbindung mit dem Magen-DarmTrakt, von Erkrankungen im Zusammenhang mit dem endokrinen System, von Erkrankungen im Zusammenhang mit dem Immunsystem, von Erkrankungen im Zusammenhang mit dem zentralen Nervensystem, von Hauterkrankungen, von durch Mikroorganismen verursachten Erkrankungen und von Krebserkrankungen sowie zur präventiven und/oder kurativen Behandlung von Diabetes.

23. Zusammensetzung in Form eines thermogeformten Extrudats, das gemäß dem Verfahren nach irgendeinem der Ansprüche 16 bis 21 erhalten wird, wobei die Zusammensetzung mindestens ein Triterpen und/oder mindestens ein Triterpenoid und/oder mindestens eine ihrer glykosylierten Formen als Wirkstoff und mindestens ein Polymer, das aus der Gruppe ausgewählt ist, die aus natürlichen oder synthetischen Proteinen, natürlichen oder synthetischen Oligosacchariden, natürlichen oder synthetischen Polysacchariden und deren Mischungen besteht, umfasst, wobei das mindestens eine Triterpen und/oder das mindestens eine Triterpenoid und/oder die mindestens eine ihrer glykosylierten Formen mindestens eine erste amorphe Phase und gegebenenfalls eine zweite kristalline Phase umfasst.

## Claims

1. A composition in the form of a thermoformed extrudate, eventually packaged in the form of pellets, flakes, granules, powders, effervescent or non-effervescent tablets, injectable or non-injectable solutions, suspensions, gels, ointments or even in any other suitable form allowing administration to an animal or a human being, comprising at least one triterpene and/or at least one triterpenoid and/or at least one of the glycosylated forms thereof and at least one polymer selected from the group consisting of natural or synthetic proteins, natural or synthetic oligosaccharides, natural or synthetic polysaccharides, and the mixtures thereof, said at least one triterpene and/or said at least one triterpenoid and/or said at least one of the glycosylated forms thereof comprising at least one first amorphous phase and optionally one second crystalline phase.

2. The composition according to claim 1, **characterized in that** said thermoformed extrudate comprises a thermoformed mixture of said at least one triterpene and/or of said at least one triterpenoid and/or of said at least one of the glycosylated forms thereof and of said at least one polymer selected from the group consisting of natural or synthetic proteins, natural or synthetic oligosaccharides, natural or synthetic polysaccharides, and the mixtures thereof.

3. The composition according to claim 1 or 2, **characterized in that** said at least one triterpene and/or said at least one triterpenoid and/or said at least one of the glycosylated forms thereof predominantly comprises at least one first amorphous phase.

4. The composition according to claim 3, **characterized in that** said at least one triterpene and/or said at least one triterpenoid and/or said at least one of the glycosylated forms thereof comprises between 51 and 100% by mass of an amorphous phase and between 0 and 49% by mass of a crystalline phase.

5. The composition according to any one of the preceding claims, **characterized in that** said at least one triterpene and/or said at least one triterpenoid and/or said at least one of the glycosylated forms thereof is tetracyclic, such as for example oleandrine, euphol or cucurbitacin, or pentacyclic, such as for example betulinic acid, oleanolic acid, boswellic acid, ursolic acid, lupeol, asiatic acid, madecassic acid, maslinic acid, jujubogenin or pseudojujubogenin.

6. The composition according to claim 5, **characterized in that** said boswellic acid is selected from the group consisting of α- and β-boswellic acids, for example α-boswellic acid, acetyl-α-boswellic acid, β-boswellic acid, acetyl-β-boswellic acid, 9,11-dehydro-α-boswellic acid, acetyl-9,11-dehydro-α-boswellic acid, 9,11-dehydro-β-boswellic acid, acetyl-9,11-dehydro-β-boswellic acid, 11-keto-β-boswellic acid, 11-keto-α-boswellic acid, 3-acetyl-11-keto-α-boswellic acid and 3-acetyl-11-keto-β-boswellic acid.

7. The composition according to any one of the preceding claims, **characterized in that** said natural or synthetic proteins are selected from the group consisting of glycoproteins, collagens and/or collagen hydrolysates, plant proteins, animal proteins, the derivatives thereof, and the mixtures thereof.

8. The composition according to claim 7, **characterized in that** said collagens and/or said collagen hydrolysates have a molecular weight comprised between 50 and 300000 Da, preferably between 100 and 275000 Da, preferably between 150 and 250000 Da, preferably between 200 and 225000 Da, preferably between 250 and 200000 Da, preferably between 300 and 175000 Da, preferably between 350 and 150000 Da, preferably between 400 and 125000 Da, preferably between 450 and 100000 Da, preferably between 500 and 75000 Da, preferably between 550 and 50000 Da, preferably between 600 and 40000 Da, preferably between 650 and 30000 Da, preferably between 700 and 20000 Da, preferably between 750 and 10000 Da, preferably between 800 and 9000 Da, preferably between 850 and 8000 Da, preferably between 900 and 7000 Da, preferably between 950 and 6000 Da, preferably between 1000 and 5000 Da, preferably between 1050 and 4000 Da, preferably between 1100 and 3000 Da, preferably between 1150 and 2000 Da, preferably between 1200 and 1000 Da.

9. The composition according to any one of the preceding claims, **characterized in that** said oligosaccharides are selected from the group consisting of cyclodextrin, raffinose, rhamminose, rhamnose, stachyose, verbascose, trehalose, lactose, lactulose, maltose, the derivatives thereof, and the mixtures thereof.

10. The composition according to any one of the preceding claims, **characterized in that** said natural or synthetic polysaccharides are selected from the group consisting of starches, fibers, celluloses, hemicelluloses, glycogen, β-glucan, inulin, amylopectin, amylose, dextrin, maltodextrin, isomaltose, xylan, pullulan, agar-agar, carrageenans, mannans, fucoidan, gums, chitosan, chitin, xanthan, levan, neoserine, hyaluronic acid, hyaluronates, chondroitin sulphate, dermatan sulphate, keratan sulphate, the derivatives thereof, and the mixtures thereof.

11. The composition according to any one of the preceding claims, **characterized in that** it further comprises at least one additional natural or synthetic polymer selected from the group consisting of polyvinyl acetate, polyvinylpyrrolidone (PVP), polyvinylpyrrolidone-co-vinyl acetate, polyethylene-co-vinyl acetate, polyvinyl acid co-methacrylic acetate, polyethylene oxide, polylactide-co-glycolide, polyvinyl alcohol, polycarbophil, polycaprolactone, carnauba wax, ethylene-vinyl copolymer, lecithin, castor oil, hydrogenated soybean oil, waxes, isomalt, the derivatives thereof, and the mixtures thereof.

12. The composition according to any one of the preceding claims, **characterized in that** it further comprises at least one plasticizer.

13. The composition according to claim 12, **characterized in that** said at least one plasticizer is selected from the group consisting of polyols, lipids, sucrose esters, water, triethyl citrate, polyethylene glycol, dibutyl sebate, butyl stearate, glycerol monostearate, diethyl phthalate, the derivatives thereof, and the mixtures thereof.

14. The composition according to any one of the preceding claims, **characterized in that** it further comprises at least one additive selected from the group consisting of lubricants, surfactants, antioxidants, chelants, the derivatives thereof, and the mixtures thereof.

15. The composition according to any one of the preceding claims, **characterized in that** it further comprises at least one first additional compound of polyphenol type selected from the group consisting of phenolic acids, stilbenes, phenolic alcohols, lignans, flavonoids, the derivatives thereof, and the mixtures thereof.

16. A manufacturing method, in particular a method for manufacturing by thermoforming, a composition in the form of a thermoformed extrudate according to any one of claims 1 to 15, **characterized in that** it comprises the following steps:
a) a step of simultaneous or delayed supplying at least one triterpene and/or at least one triterpenoid and/or at least one of the glycosylated forms thereof and at least one polymer selected from the group consisting of natural or synthetic proteins, natural or synthetic oligosaccharides, natural or synthetic polysaccharides, and the mixtures thereof, to be fed into an extruder,
b) a step of mixing, in said extruder, said at least one triterpene and/or said at least one triterpenoid and/or said at least one of the glycosylated forms thereof and said at least one polymer selected from the group consisting of natural or synthetic proteins, natural or synthetic oligosaccharides, natural or synthetic polysaccharides, and the mixtures thereof, to form a mixture, and
c) a step of hot extruding said mixture obtained in step b) in said extruder to obtain a thermoformed extrudate in which said at least one triterpene and/or said at least one triterpenoid and/or said at least one of the glycosylated forms thereof comprises/comprise at least one first amorphous phase and optionally one second crystalline phase.

17. The method according to claim 16, **characterized in that** it comprises a preliminary step of premixing said at least one triterpene and/or said at least one triterpenoid and/or said at least one of the glycosylated forms thereof and said at least one polymer selected from the group consisting of natural or synthetic proteins, natural or synthetic oligosaccharides, natural or synthetic polysaccharides, and the mixtures thereof, in such a way to form a premixture intended to be fed into the extruder.

18. The method according to claim 16 or 17, **characterized in that** said hot extrusion step is carried out at an extrusion temperature comprised between 20 and 300°C, preferably at a temperature comprised between 40 and 270°C, preferentially at a temperature comprised between 50 and 250°C, preferably at a temperature comprised between 60 and 230°C, more preferentially at a temperature comprised between 70 and 220°C, more preferentially at a temperature comprised between 80 and 200°C, more preferentially at a temperature comprised between 90 and 180°C, more preferentially at a temperature comprised between 100 and 170°C, more preferentially at a temperature comprised between 120 and 160°C, more preferentially at a temperature comprised between 125 and 150°C.

19. The method according to any one of claims 16 to 18, **characterized in that** said hot extrusion step is carried out at a rotation speed of an extrusion screw comprised between 20 and 900 rpm, preferably comprised between 50 and 300 rpm, preferably comprised between 100 and 250 rpm, preferentially equal to 250 rpm, preferentially equal to 100 rpm.

20. The method according to any one of claims 16 to 19, **characterized in that** it comprises an additional step of cooling at the outlet of the extruder6

21. The method according to any one of claims 16 to 20, **characterized in that** it comprises an additional step of treating the thermoformed extrudate at the outlet of the extruder, for example cutting at a pelletizer and/or grinding said thermoformed extrudate.

22. The composition in the form of a thermoformed extrudate according to any one of claims 1 to 15 for use in the preventive and/or curative treatment, in human and/or in animal, of pathologies related to inflammations, pathologies related to the premature aging of cells, pathologies related to the cardiovascular system, pathologies related to the blood system, pathologies related to the gastrointestinal system, pathologies related to the endocrine system, pathologies related to the immune system, pathologies related to the central nervous system, skin diseases, diseases due to the presence of microorganisms and cancers and in the preventive and/or curative treatment of diabetes.

23. The composition in the form of a thermoformed extrudate obtained according to the method according to any one of claims 16 to 21, said composition comprising at least one triterpene and/or at least one triterpenoid and/or at least one of the glycosylated forms thereof such as active ingredient and at least one polymer selected from the group consisting of natural or synthetic proteins, natural or synthetic oligosaccharides, natural or synthetic polysaccharides, and the mixtures thereof, said at least one triterpene and/or said at least one triterpenoid and/or said at least one of the glycosylated forms thereof comprising at least one first amorphous phase and optionally one second crystalline phase.
